(19) Europäisches Patentamt / European Patent Office / Office européen des brevets

(11) **EP 4 139 325 B1**

(12) **EUROPEAN PATENT SPECIFICATION**

(45) Date of publication and mention of the grant of the patent:
**17.06.2026 Bulletin 2026/25**

(21) Application number: **21724482.1**

(22) Date of filing: **21.04.2021**

(51) International Patent Classification (IPC):
***G01N 30/86*** (2006.01) ***C07K 1/16*** (2006.01)
***B01D 15/08*** (2006.01) ***G16B 40/20*** (2019.01)
***G16C 20/10*** (2019.01) ***G16C 20/70*** (2019.01)

(52) Cooperative Patent Classification (CPC):
**G01N 30/8662; B01D 15/08; C07K 1/16; G01N 30/8693; G16B 40/20**

(86) International application number:
**PCT/US2021/028291**

(87) International publication number:
**WO 2021/216635 (28.10.2021 Gazette 2021/43)**

(54) **SELECTING CHROMATOGRAPHY PARAMETERS FOR MANUFACTURING THERAPEUTIC PROTEINS**

AUSWAHL VON CHROMATOGRAPHIEPARAMETERN ZUR HERSTELLUNG THERAPEUTISCHER PROTEINE

SÉLECTION DE PARAMÈTRES CHROMATOGRAPHIQUES POUR FABRIQUER DES PROTÉINES THÉRAPEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2020 US 202063014273 P**

(43) Date of publication of application:
**01.03.2023 Bulletin 2023/09**

(73) Proprietor: **Amgen Inc.**
**Thousand Oaks, CA 91320-1799 (US)**

(72) Inventors:
- **DAN, Or**
  **Thousand Oaks, CA 91320 (US)**
- **HART, Roger**
  **Thousand Oaks, CA 91320 (US)**
- **WONG, Hann-chung**
  **Thousand Oaks, CA 91320 (US)**

(74) Representative: **Gillott-Jones, Nathan Philip et al**
**Kilburn & Strode LLP**
**Lacon London**
**84 Theobalds Road**
**London WC1X 8NL (GB)**

(56) References cited:
**WO-A1-2017/174580 WO-A1-2019/165148**
**WO-A2-2006/083262**

- **RYAN K SWANSON ET AL: "Proteomics-based, multivariate random forest method for prediction of protein separation behavior during cation-exchange chromatography", JOURNAL OF CHROMATOGRAPHY A, ELSEVIER, AMSTERDAM, NL, vol. 1249, 3 June 2012 (2012-06-03), pages 103 - 114, XP028401569, ISSN: 0021-9673, [retrieved on 20120612], DOI: 10.1016/J.CHROMA.2012.06.009**
- **TARASOVA IRINA A. ET AL: "Predictive chromatography of peptides and proteins as a complementary tool for proteomics", ANALYST, vol. 141, no. 16, 1 January 2016 (2016-01-01), UK, pages 4816 - 4832, XP055822528, ISSN: 0003-2654, DOI: 10.1039/C6AN00919K**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SONG MINGHU ET AL: "Prediction of Protein Retention Times in Anion-Exchange Chromatography Systems Using Support Vector Regression", JOURNAL OF CHEMICAL INFORMATION AND COMPUTER SCIENCES., vol. 42, no. 6, 1 November 2002 (2002-11-01), US, pages 1347 - 1357, XP055822406, ISSN: 0095-2338, DOI: 10.1021/ci025580t**
- **LIU SONGSONG ET AL: "Optimal Antibody Purification Strategies Using Data-Driven Models", ENGINEERING, vol. 5, no. 6, 1 December 2019 (2019-12-01), pages 1077 - 1092, XP055822488, ISSN: 2095-8099, DOI: 10.1016/ j.eng.2019.10.011**
- **SWANSON RYAN K. ET AL: "Accounting for host cell protein behavior in anion-exchange chromatography", BIOTECHNOLOGY PROGRESS, vol. 32, no. 6, 21 October 2016 (2016-10-21), pages 1453 - 1463, XP055822518, ISSN: 8756-7938, Retrieved from the Internet <URL:https://onlinelibrary.wiley.com/doi/ full-xml/10.1002/btpr.2342> DOI: 10.1002/ btpr.2342**
- **BUYEL J F ET AL: "The use of quantitative structure-activity relationship models to develop optimized processes for the removal of tobacco host cell proteins during biopharmaceutical produc", JOURNAL OF CHROMATOGRAPHY A, ELSEVIER, AMSTERDAM, NL, vol. 1322, 30 October 2013 (2013-10-30), pages 18 - 28, XP028790413, ISSN: 0021-9673, DOI: 10.1016/ J.CHROMA.2013.10.076**
- **SUSANTO A. ET AL: "High Throughput Screening for the Design and Optimization of Chromatographic Processes: Automated Optimization of Chromatographic Phase Systems", CHEMICAL ENGINEERING & TECHNOLOGY, vol. 32, no. 1, 1 January 2009 (2009-01-01), pages 140 - 154, XP055822660, ISSN: 0930-7516, DOI: 10.1002/ceat.200800350**

## Description

### FIELD OF DISCLOSURE

The present application relates generally to the production of biopharmaceutical products, and more specifically relates to techniques for modeling a chromatography process (such as a chromatography purification process) to facilitate the selection of chromatography parameters when manufacturing therapeutic proteins.

### BACKGROUND

In the biopharmaceutical industry, large, complex protein molecules known as biologics or therapeutic proteins are derived from living systems. At a high level, the process of manufacturing the therapeutic proteins includes the following steps: (1) a host cell selection stage, in which the master cell line containing the gene that makes the desired protein is produced (e.g., using Chinese hamster ovary (CHO) cells); (2) a cell culture stage, in which defined culture media are used to grow large numbers of cells that produce the protein in bioreactors; (3) a purification stage, in which the recovery and purification of the product from the previous stage is performed to isolate the protein; and (4) a formulation and fill-finish-package stage, in which the protein is prepared for use by physicians or patients.

FIG. 2 depicts a typical therapeutic protein manufacturing process 10. At a first stage 12, after an optimal cell that produces high concentrations of the desired protein is engineered and cryopreserved in vials or cell bags, an "upstream" manufacturing process is initiated by reviving the cryopreserved cells. The cells are typically thawed into small T-flasks, shake flasks, or spinner flasks, and expanded in increasing numbers and increasing flask sizes to achieve inoculation of a seed bioreactor at stage 14. Throughout the expansion process, the cells are kept in controlled conditions (e.g., temperature, pH, and/or nutrients) for continued growth. Following one or more stages of culture volume expansion (denoted as stage 16 in FIG. 2), the cells are inoculated into a production bioreactor at stage 18. During stage 18, the therapeutic protein is expressed by the cells. Following this step, a "downstream" process begins. In the downstream process, centrifugation or depth filtration is performed at stage 20 to separate the culture medium from cells and/or separate the desired protein from other molecules in the bioreactor. At stage 22, a chromatographic purification process further isolates the desired protein from the host cells and impurities or other undesired matter (e.g., degraded or aggregated proteins, etc.). Various filtering technologies may be used at stage 22 to isolate and purify the proteins based on their size, molecular weight, and electrical charge. The resulting substance undergoes virus filtration at stage 24. The purified protein is typically formulated with an excipient to produce a sterile solution that can be injected or infused. At stage 26, the substance is concentrated and placed in a target buffer, yielding a formulation which is placed within containers (e.g., vials or syringes) for labeling, long-term storage, and shipment. This example therapeutic manufacturing process 10 is provided for illustrative purposes, but it will be appreciated that the selection of chromatography parameters described herein can readily be applied to other therapeutic protein manufacturing processes that include chromatography.

In general, "chromatography" (e.g., as performed at stage 22) refers to a separation process wherein molecules are distributed between two phases: (1) a stationary phase, which is often a chromatography resin; and (2) a mobile phase, which in the case of protein separation is a solvent, such as water or chloroform. Molecules that are more strongly attracted to the stationary phase move more slowly through the system as compared to those that are more strongly attracted to the mobile phase. For commercial manufacturing purification, chromatography is typically carried out as column chromatography due to scale considerations. In a common chromatographic operation, a volume of sample is injected into the column. Eluent is then pumped through the column, causing molecules to be separated based on their relative affinity for the stationary resin and the eluent. Different molecules will elute from the column at different times and after different volumes of eluent have passed through the column. Accordingly, therapeutic proteins can be separated from other substances that elute from the column at different times. This information is captured in a chromatogram, which is a plot of the concentration exiting the column versus time.

Hydrophobic interaction chromatography can be used to separate proteins based on differences in their hydrophobicity, affinity chromatography can be used to separate molecules based on differences in their affinity for a target ligand attached to a chromatography resin, and ion exchange chromatography can be used to separate molecules based on differences in molecular charge. As a more specific example, cation-exchange chromatography (CEX) is an ion exchange chromatography used when the molecule of interest is positively charged. Proteins have amino acids with acidic and basic side chains. Depending on the acidity level (pH) of the solution surrounding the biologic, the molecule can be positively charged, negatively charged, or neutral. The isoelectric point (pl) is the pH at which the number of protonated and deprotonated groups is equal, and the protein has no net charge. If the pH is greater than the pl, a protein will have a net negative charge, and if the pH is less than the pl, the protein will have a net positive charge. Because the pl of a protein is determined by the primary amino acid sequence of the protein, and can thus be calculated, a buffer can be chosen that ensures a known net charge for a protein of interest. Proteins with different pl values will have varying degrees of charge at a given pH, and so different proteins will bind to the resin with different strengths, facilitating their separation through the column. Other common types of chromatography include size-exclusion chromatography (SEC), in which molecules in solution are separated by size and/or molecular weight, and Protein A chromatography.

Conventionally, selecting chromatography parameters (e.g., elution buffer pH, elution buffer conductivity, elution buffer molarity, gradient slope, linear velocity, load, and collection times), and determining how the purification stage will perform for a particular product/molecule (e.g., with a particular solution, at a particular pH, etc.) can be highly resource-intensive in terms of time, cost, labor, and usage of equipment, and can require a great deal of trial and error by setting up and running numerous experiments to obtain empirical measurements. As the pace of biotechnology quickens, however, and as an increased emphasis is placed on processing additional molecules in the pipeline, there is an increasing need to more quickly design and implement manufacturing processes, including the chromatographic purification process.

TARASOVA IRINA A. ET AL, "Predictive chromatography of peptides and proteins as a complementary tool for proteomics", ANALYST, UK, (20160101), vol. 141, no. 16, doi:10.1039/C6AN00919K, ISSN 0003-2654, pages 4816 - 4832, discloses computational and theoretical models for the prediction of peptide retention time for a given sequence. SONG MINGHU ET AL, "Prediction of Protein Retention Times in Anion-Exchange Chromatography Systems Using Support Vector Regression", JOURNAL OF CHEMICAL INFORMATION AND COMPUTER SCIENCES., US, (20021101), vol. 42, no. 6, doi:10.1021/ci025580t, ISSN 0095-2338, pages 1347 - 1357 discloses Quantitative Structure-Retention Relationship (QSRR) models for the prediction of protein retention times in anion-exchange chromatography systems. Topological, subdivided surface area, and TAE (Transferable Atom Equivalent) electron-density-based descriptors are computed directly for a set of proteins using molecular connectivity patterns and crystal structure geometries. A novel algorithm based on Support Vector Machine (SVM) regression is employed to obtain predictive QSRR models using a two-step computational strategy. LIU SONGSONG ET AL, "Optimal Antibody Purification Strategies Using Data-Driven Models", ENGINEERING, (20191201), vol. 5, no. 6, doi:10.1016/j.eng.2019.10.011, ISSN 2095-8099, pages 1077 - 1092 discloses data-driven models of chromatography throughput considering loaded mass, flow velocity, and column bed height as the inputs, using manufacturing-scale simulated datasets based on microscale experimental data. A piecewise linear regression modeling method is adapted due to its simplicity and better prediction SWANSON RYAN K. ET AL, "Accounting for host cell protein behavior in anion-exchange chromatography", BIOTECHNOLOGY PROGRESS, vol. 32, no. 6, doi:10.1002/btpr.2342, discloses characterizing the separation behavior of complex mixtures of HCP during anion-exchange chromatography (AEX), A multivariate random forest (MVRF) statistical methodology was then applied to a database of characterized proteins creating a tool for predicting the AEX behavior of a mixture of proteins. RYAN K SWANSON ET AL: "Proteomics-based, multivariate random forest method for prediction of protein separation behavior during cation-exchange chromatography", JOURNAL OF CHROMATOGRAPHY A, vol. 1249, DOI: 10.1016/J.CHROMA.2012.06.009 discloses the use of a multivariate random forest model for prediction of protein separation behaviour during cation-exchange chromatography. WO 2017/174580 A1 discloses predicting concentration, purity and/or potency of a biological product to be purified using random forests and elastic net machine learning models.

## SUMMARY

The invention is set out in the appended claims.

Embodiments described herein relate to systems and methods that create and apply one or more models that are predictive of performance of a purification process in the manufacture of therapeutic proteins. The therapeutic proteins may be any suitable type of protein, such as a monoclonal antibody ("mAb") or a bispecific or other multi-specific antibody, for example. More specifically, in these embodiments, machine leaming models are used to predict performance indicators (e.g., product yield and/or quality metrics) of a chromatography purification process, such as a CEX, SEC, Protein A, or any other suitable chromatography process, based on various process parameters (e.g., buffer and/or elution buffer and/or load pH, elution buffer molarity, elution buffer conductivity, gradient slope, linear velocity, load conductivity, load factor, stop collect, column volume, actual CEX loading (if CEX is used), loading flow rate, elution flow rate, buffer concentration, gradient length, gradient start, gradient end, pool volume, protein concentration, pool start, and/or pool end) and molecular descriptors (e.g., mathematical representations of physical characteristics of a molecule). The process may result in a better selection of chromatography process parameters as compared to conventional processes, a substantial reduction in the amount of time required to design/develop/implement the downstream manufacture process (e.g., by obviating or reducing the need to conduct experiments), and/or a substantial reduction in the usage of other resources (e.g., labor, equipment, costs). Moreover, the process may reveal how different molecule physical characteristics (relating to various molecule descriptors) affect chromatography performance, thereby providing insights into molecule design.

It is contemplated that performance indicators are typically dependent on process parameters. As described herein, non-null process parameters can be predicted based on one or more performance indicator, permitting the efficient prediction of process parameters (or accuracy ranges thereof) based on one or more desired performance indcators. Some embodiments herein relate to systems and methods that create and apply one or more models that facilitate selection of chromatography parameters for a purification process based on one or more desired performance indicators.

These methods may be used for facilitating selection of chromatography parameters for a purification process during manufacture of a therapeutic protein. In these embodiments, machine learning models are used to predict process parameters (e.g., buffer and/or elution buffer and/or load pH, elution buffer molarity, elution buffer conductivity, gradient slope, linear velocity, load conductivity, load factor, stop collect, column volume, actual CEX loading (if CEX is used), loading flow rate, elution flow rate, buffer concentration, gradient length, gradient start, gradient end, pool volume, protein concentration, pool start, and/or pool end) based on one or more performance indicator (e.g., product yield and/or quality metrics) of a chromatography purification process, such as a CEX, SEC, Protein A, or any other suitable chromatography process, based on various process parameters and molecular descriptors (e.g., mathematical representations of physical characteristics of a molecule).

Moreover, interpretable machine learning algorithms may be used to identify the input features (e.g., molecular descriptors and process parameters or performance indicators) that are most important to generating accurate predictions. This can be particularly helpful given that the number of process parameters, performance indicators, and especially the number of potential molecular descriptors, can be vast (e.g., hundreds or even thousands of potential molecular descriptors). Thus, for example, it may be possible to make sufficiently accurate predictions for the purification process using a relatively small number of input features, and eliminating the need to measure or calculate numerous other parameters and/or descriptors. Knowledge of the correlations between input parameters/descriptors and prediction targets can also provide scientific insight, and spawn hypotheses for further investigation that can lead to future bioprocess improvements.

## BRIEF DESCRIPTION OF THE DRAWINGS

The skilled artisan will understand that the figures, described herein, are included for purposes of illustration and do not limit the present disclosure. The drawings are not necessarily to scale, and emphasis is instead placed upon illustrating the principles of the present disclosure. It is to be understood that, in some instances, various aspects of the described implementations may be shown exaggerated or enlarged to facilitate an understanding of the described implementations. In the drawings, like reference characters throughout the various drawings generally refer to functionally similar and/or structurally similar components.

FIG. 1 is a simplified block diagram of an example system that may implement the techniques described herein.

FIG. 2 depicts a prior art process for manufacturing a drug substance.

FIG. 3 is a flow diagram of an example process for generating a machine learning model for use in the system of FIG. 1.

FIGs. 4A and 4B depict example feature importance metrics for predicting experimental yield or SE-HPLC HMW using an eXtreme gradient boost model.

FIGs. 5A and 5B are flow diagrams of example methods for facilitating selection of chromatography parameters for a purification process during the manufacture of therapeutic proteins.

## DETAILED DESCRIPTION

The various concepts introduced above and discussed in greater detail below may be implemented in any of numerous ways, and the described concepts are not limited to any particular manner of implementation. Examples of implementations are provided for illustrative purposes.

FIG. 1 is a simplified block diagram of an example system 100 that may implement the techniques described herein. System 100 includes a computing system 102 communicatively coupled to a training server 104 via a network 106. Generally, computing system 102 and/or training server 104 are configured to train one or more machine learning (ML) models 108, and use the trained model(s) to predict performance (e.g., yield and/or product quality metrics) for hypothetical chromatography processes that can be used in the manufacture of therapeutic proteins. It should be appreciated that the term "hypothetical," as used herein, does not necessarily mean that no corresponding, real-world process exists. For example, predicted performance may be compared with measured performance by running one of ML model(s) 108 in parallel with, or even after, a corresponding real-world chromatography purification process. The chromatography purification process may include at least one of a CEX process, an SEC process, a Protein A chromatography process, or any other suitable chromatography process.

The ML model(s) 108 may predict performance based on process parameters (e.g., elution buffer pH, salt concentration, column volume, etc.), molecular descriptors (e.g., parameters including or relating to molecule charge, hydrophobicity, isoelectric point, dipole moment, etc.), and/or other numerical and/or categorical parameters (e.g.,

modality, such as monoclonal antibody (mAb)) or bispecific antibody, etc.). Computing system 102 is also generally configured to enable one or more users, who may be local or remotely distributed, to make use of the prediction capabilities of computing system 102, and to provide various interactive capabilities to the user(s) as discussed elsewhere herein.

Network 106 may be a single communication network, or may include multiple communication networks of one or more types (e.g., one or more wired and/or wireless local area networks (LANs), and/or one or more wired and/or wireless wide area networks (WANs) such as the Internet). In various embodiments, training server 104 may train and/or utilize ML model(s) 108 as a "cloud" service (e.g., Amazon Web Services), or training server 104 may be a local server. In the depicted embodiment, however, ML model(s) 108 is/are trained by server 104, and then transferred to computing system 102 via network 106 as needed. In other embodiments, one, some or all of ML model(s) 108 may be trained on computing system 102, and then uploaded to server 104. In still other embodiments, computing system 102 trains and maintains/-stores the model(s) 108, in which case system 100 may omit both network 106 and training server 104, or server 104 may be a part of computing system 102.

Computing system 102 may include one or more general-purpose computers specifically programmed to perform the operations discussed herein, and/or may include one or more special-purpose computing devices. As seen in FIG. 1, computing system 102 includes a processing unit 120, a network interface 122, a display 124, a user input device 126, and a memory unit 128. In embodiments where computing system 102 includes two or more computers (either co-located or remote from each other), the operations described herein relating to at least processing unit 120, network interface 122, and/or memory unit 128 may be divided among multiple processing units, multiple network interfaces, and/or multiple memory units, respectively. Moreover, display 124 and user input device 126, while referred to herein in the singular, may include multiple displays and multiple user input devices, respectively. For example, display 124 may include at least one display at each of a number of remote, user-specific client devices, and user input device 126 may include at least one user input device for each of those client devices.

Processing unit 120 includes one or more processors, each of which may be a programmable microprocessor that executes software instructions stored in memory unit 128 to execute some or all of the functions of computing system 102 as described herein. Processing unit 120 may include one or more central processing units (CPUs) and/or one or more graphics processing units (GPUs), for example. Alternatively, or in addition, some of the processors in processing unit 120 may be other types of processors (e.g., application-specific integrated circuits (ASICs), field-programmable gate arrays (FPGAs), etc.), and some of the functionality of computing system 102 as described herein may instead be implemented in hardware.

Network interface 122 may include any suitable hardware (e.g., a front-end transmitter and receiver hardware), firmware, and/or software configured to communicate with training server 104 via network 106 using one or more communication protocols. For example, network interface 122 may be or include an Ethernet interface, enabling computing system 102 to communicate with training server 104 over the Internet or an intranet, etc.

Display 124 may use any suitable display technology (e.g., LED, OLED, LCD, etc.) to present information to a user, and user input device 126 may be a keyboard or other suitable input device. In some embodiments, display 124 and user input device 126 are integrated within a single device (e.g., a touchscreen display). Generally, display 124 and user input device 126 may combine to enable a user to interact with graphical user interfaces (GUIs) provided by computing system 102. However, computing system 102 may omit display 124 and/or user input device 126, e.g., in certain embodiments where computing system 102 interacts with other computing devices or systems (e.g., client devices of third parties) to enable interaction by users of those devices or systems.

Memory unit 128 may include one or more volatile and/or non-volatile memories. Any suitable memory type or types may be included, such as read-only memory (ROM), random access memory (RAM), flash memory, a solid-state drive (SSD), a hard disk drive (HDD), and so on. Collectively, memory unit 128 may store one or more software applications, the data received/used by those applications, and the data output/generated by those applications. These applications include a chromatography modeling application 130 that, when executed by processing unit 120, predicts performance (e.g., yield and/or quality metrics) of a hypothetical chromatography process for purification during therapeutic protein manufacture. In some embodiments, the various "units" of application 130 discussed herein may be distributed among different software applications, and/or the functionality of any one such unit may be divided among two or more software applications.

In the example system 100, application 130 includes a data collection unit 132, a prediction unit 134, and a visualization unit 136. In general, data collection unit 132 receives (e.g., retrieves) the parameters that prediction unit 134 applies as inputs to a local machine learning (ML) model 138, to predict a performance indicator (e.g., a yield or product quality metric) or a process parameter (or accuracy range thereof). In the depicted embodiment, ML model 138 is a local copy of one of the model(s) 108 trained by training server 104, and may be stored in a RAM of memory unit 128, for example. As noted above, however, server 104 may utilize/run all models 108 in some embodiments, in which case no local copy need be present in memory unit 128, or all of model(s) 108 may reside in a persistent memory of memory unit 128 rather than being retrieved from training server 104 on an as-needed basis. Data collection unit 132 may receive the values from a user entering parameters/values via a GUI (e.g., on display 124) that is generated or populated by visualization unit 136, and/or may receive values as one or more files or other data transfers (e.g., using file paths designated by a user via such a GUI), for example.

Visualization unit 136 may also generate and/or populate a GUI to view and/or interact with the predicted results of that modeled process (e.g., values of the performance indicator output or process parameter output by prediction unit 134 using model 138), for example. Depending on the embodiment, visualization unit 136 may also provide tools for users to develop useful models (e.g., identify the most predictive features for a given performance indicator or process parameter, optimize hyperparameters for the model, etc.), and/or for users to make use of such models when designing (e.g., optimizing) a chromatography purification process (e.g., to achieve a process with high yield and good product quality attributes, and/or a process that is highly consistent/repeatable, etc.).

In the example system 100, memory unit 128 also stores software instructions of a molecular operating environment (MOE) application 139 that provides homology modeling for therapeutic proteins of interest. Generally, MOE application 139 is configured to generate descriptors for a molecule (e.g., mathematical representations of physical characteristics of the molecule, such as charge, hydrophobicity, dipole moment, isoelectric point, etc.) based on input information about the molecule. For example, a user may use MOE application 139 to enter the amino acid sequence of the molecule (e.g., via user input device 126), and to select an appropriate molecule template. MOE application 139 may then attempt to "fit" the amino acid sequence to the selected template. Alternatively, or in addition, MOE application 139 may generate the descriptors based on experimental/measured results for the molecule. In alternative embodiments, MOE application 139 is stored and executed by a computing device or system other than computing system 102 (e.g., by a third party computing device or system).

Operation of system 100, according to one embodiment, will now be described in further detail. Initially, training server 104 trains ML model(s) 108 using historical data stored in a training database 140. Training database 140 includes multiple databases stored in one or more memories. ML model(s) 108 include a number of different types of machine learning models, including an eXtreme gradient boost (or "xgboost") model, a regression (or "decision" or "ID") tree model, an elastic net model, and may further include a lasso model, a ridge model, a stochastic gradient descent (SGD) regularized loss linear model, a linear support vector machine (SVM) model, a partial least squares (PLS) regression model, and/or one or more other suitable model types. Moreover, different models of ML models 108 are trained to predict different performance indicators (e.g., yield, specific CEX readings, specific SEC readings, etc.) or different process parameters (e.g., buffer and/or elution buffer and/or load pH, elution buffer molarity, elution buffer conductivity, gradient slope, linear velocity, load conductivity, load factor, stop collect, column volume, actual CEX loading (if CEX is used), loading flow rate, elution flow rate, buffer concentration, gradient length, gradient start, gradient end, pool volume, protein concentration, pool start, and/or pool end). ML models 108 specifically include a regression tree model for predicting a first set of one or more performance indicators, an xgboost model for predicting a different, second set of one or more performance indicators, and an elastic net model for predicting a different, third set of one or more performance indicators. ML models 108 specifically include a regression tree model for predicting a first set of one or more process parameters, an xgboost model for predicting a different, second set of one or more process parameters, and an elastic net model for predicting a different, third set of one or more process parameters. Further, in some embodiments, ML model(s) 108 may include more than one model of any given type (e.g., two or more models of the same type that are trained on different historical datasets, using different feature sets, and/or having different hyperparameters). In some embodiments, and as discussed in further detail in connection with FIGs. 4A and 4B, each of ML model(s) 108 may be used to identify which features (e.g., process parameters, molecular descriptors, etc.) are most predictive of a particular performance indicator (as applicable), which features (e.g., performance indicators, molecular descriptors, etc.) are most predictive of a process parameter (as applicable), and/or may be trained or re-trained using a feature set that only includes the features that are most predictive of a particular performance indicator or process parameter.

For each different model within ML model(s) 108, training database 140 may store a corresponding set of training data (e.g., input/feature data, and corresponding labels), with possible overlap between the training data sets. To train a model that predicts yield percentage, for instance, training database 140 may include numerous sets of inputs/features each comprising historical process parameters (e.g., pH levels, loading flow rates, salt concentrations, etc.), which may have been made by analytical instruments, as well as molecular descriptors (e.g., descriptors relating to charge, hydrophobicity, isoelectric point, etc.) calculated by software (e.g., by MOE application 139 or similar software) for the protein that was being manufactured, and possibly other information (e.g., modality of the protein that was being manufactured), along with a label for each feature set. In this example, the label for each feature set indicates the yield percentage that was measured when the particular therapeutic protein was purified in the chromatography process. In some embodiments, all features and labels are numerical, with non-numerical classifications or categories being mapped to numerical values (e.g., with the allowable values [Monoclonal, Bispecific Format 1, Bispecific Format 2, Bispecific Format 1 or 2] of a modality feature/input being mapped to the values [00, 10, 01, 11]).

In some embodiments, training server 104 uses additional labeled data sets in training database 140 in order to validate the trained ML model(s) 108 (e.g., to confirm that a given one of ML model(s) 108 provides at least some minimum acceptable accuracy). In some embodiments, training server 104 also updates/refines one or more of ML model(s) 108 on an ongoing basis. For example, after ML model(s) 108 is/are initially trained to provide a sufficient level of accuracy, additional measurements of chromatography process performance indicators (and corresponding input/features) may be used to improve prediction accuracy.

Application 130 may retrieve, from training server 104 via network 106 and network interface 122, a specific one of ML model(s) 108 that corresponds to a performance indicator of interest. The performance indicator may be one that was indicated by a user via a GUI that was generated or populated by visualization unit 136, for example. Upon retrieving the model, computing system 102 stores a local copy as local ML model 138. In other embodiments, as noted above, no model is retrieved, and input/feature data is instead sent to training server 104 (or another server) as needed to use the appropriate model of model(s) 108, or all of model(s) 108 may reside only at computing system 102.

The performance indicator that a particular model 108 or model 138 is trained to predict includes an indicator of an aspect of performance, such as yield or product quality (e.g., purity). Moreover, the performance indicators may be generic to different types of chromatography (e.g., yield), or may be specific to a particular type of chromatography (e.g., CEX, SEC, Protein A, etc.). For example and without limitation, an ML model 108 or 138 may predict any of the following indicators: step yield, CEX % Acidic, CEX % Main, CEX % Basic, SEC % high molecular weight (HMW), SEC % Main, SEC % low molecular weight (LMW), capillary electrophoresis sodium dodecyl sulfate with reduced sample preparation (rCE-SDS) % Main, rCE-SDS % LMW, rCE-SDS % light chain (LC) + heavy chain (HC), capillary electrophoresis sodium dodecyl sulfate without reduced sample preparation (nrCE-SDS) % Main, rCE-SDS % Pre-LC, rCE-SDS % LC, rCE-SDS % non-glycosylated heavy chain (NGHC), rCE-SDS % HC, rCE-SDS % HMW, rCE-SDS % Pre-LC+LC+HC, pool conductivity (mS/cm), capillary isoelectric focusing (cIEF) % Acidic, cIEF % Basic, cIEF % Main, nrCE-SDS % Pre-Peak, host cell protein (HCP), and SE-HPLC HMW. In some embodiments, the process parameters that a particular model 108 or model 138 is trained to predict may include an indicator of any aspect of the process, or an accuracy range thereof (for example, a confidence interval such as an 80%, 85%, 90%, or 95% confidence interval). For example and without limitation, an ML model 108 or 138 may predict any of the following process parameters: buffer and/or elution buffer and/or load pH, elution buffer molarity, elution buffer conductivity, gradient slope, linear velocity, load conductivity, load factor, stop collect, column volume, actual CEX loading (if CEX is used), loading flow rate, elution flow rate, buffer concentration, gradient length, gradient start, gradient end, pool volume, protein concentration, pool start, and/or pool end.

In accordance with the feature set used by model 138, data collection unit 132 collects the necessary data. For example, data collection unit 132 may receive user-entered process parameters (or performance indicators, as applicable), as well as molecular descriptors output by MOE application 139 for the therapeutic protein of interest (e.g., after the user enters or otherwise provides the amino acid sequence of the protein to the MOE application 139). The process parameters and performance indicators may be as described herein. The process parameters include a parameters relating to the conditions or characteristics of the hypothetical chromatography process, such as, for example and without limitation: buffer pH, elution buffer conductivity (mS/cm), elution buffer molarity (mM), elution buffer pH, gradient slope (mM/CV), linear velocity (cm/hr), load conductivity (mS/cm), load factor (g/Lr), load pH, stop collect (%), column volume, actual CEX loading, loading flow rate, elution flow rate, buffer concentration, gradient length, gradient start, gradient end, pool volume, protein concentration, pool start, and/or pool end.

The molecular descriptors generated by MOE application 139 may include any suitable descriptor types, such as, for example and without limitation, any or all of the following descriptors known to users of MOE software: pH, HI, pro_Fv_net_charge, U, asa_hyd, viscosity, hyd_idx, pro_helicity, apol, asa_hph, pro_net_charge, hyd_idx_cdr, pro_henry, b_1rotR, volume, amphipathicity, hyd_strength, pro_hyd_moment, b_rotR, mobility, ASPmax, hyd_strength_cdr, pro_mass, density, helicity, BSA, Packing Score, pro_mobility, ens_dipole, henry, BSA_HC, pro_affinity, pro_pl_3D, mass, net_charge, BSA_LC_HC, pro_app_charge, pro_pl_seq, pl_seq, app_charge, contact energy, pro_asa_hph, pro_r_gyr, pl_3D, dipole_moment, DRT, pro_asa_hyd, pro_r_solv, coeff_fric, hyd_moment, E bond, pro_asa_vdw, pro_sed_const, coeff_diff, zeta, E ele, pro_cdr_net_charge, pro_stability, r_gyr, zdipole, E sol, pro_coeff_diff, pro_volume, r_solv, zquadrupole, E vdw, pro_coeff_fric, pro_zdipole, sed_const, Eint_VL_VH, pro_dipole_moment, pro_zeta, eccen, GB/VI, pro_eccen, pro_zquadrupole, and/or asa_vdw. Preferably, in some embodiments, the molecular descriptors include at least one descriptor that is a function of (or otherwise dependent on) the pH level of the molecule's environment, and possibly many such descriptors. For example, various descriptors may depend on the surface charge of the protein molecule, and the surface charge may in turn depend on the pH of the molecule environment.

After data collection unit 132 has collected the process parameters (or performance indicators, as applicable) and molecular descriptors for a particular hypothetical chromatography process (possibly along with other data, such as a user-entered modality of the protein), prediction unit 134 causes ML model 138 to operate on those inputs/features to predict the desired performance indicator (or process parameter) for the hypothetical chromatography process. It is understood that, in some embodiments and/or scenarios, prediction unit 134 may obtain multiple, different local ML models 138 from training server 104 in order to predict (e.g., in parallel or sequentially) different performance indicators (or process parameters, as applicable) for the same hypothetical chromatography process, with the local ML models 138 operating on the same or different features to generate the respective predictions.

Visualization unit 136 causes a GUI, depicted on display 124, to present the predicted performance indicator(s) (or process parameters, as applicable), and/or other information derived from the predicted performance indicator(s) (or process parameters, as applicable). For example, visualization unit 138 may cause the GUI to present an indication of whether the predicted performance indicator(s) satisfy one or more acceptability criteria (e.g., after application 130 compares the performance indicator(s) to one or more respective threshold values). For example, visualization unit 138 may cause the GUI to present an accuracy range of the predicted process parameters.

The above prediction/visualization process may be repeated for a number of different hypothetical chromatography processes (e.g., for different combinations of process parameters with a fixed set of molecular descriptors for the therapeutic protein), thereby enabling a user to quickly test different process designs. A user can also quickly test particular aspects of a design, such as how small perturbations to a specific input (e.g., reflecting expected ranges in elution buffer pH, or loading flow rate, etc.) are likely to affect the predicted performance indicator(s). Visualization unit 136 may generate or populate one or more GUIs that help a viewing user to comprehend and consider the results of the predictions for the various hypothetical chromatography processes. In this manner, the viewing user(s) may make an informed selection of which chromatography process parameters to use in a real-world, commercial manufacturing process (subject to any necessary qualification testing). The selection of chromatography process parameters should generally attempt to maximize yield while minimizing impurities (possibly weighing one of these goals more than the other depending on the use case/project goals), and may be decided by one or more users based on the displayed information, or may be fully automated according to some predetermined selection criteria. In some cases, the techniques described herein may be used not only to select chromatography process parameters but also, or instead, to provide holistic insights into interrelations between new molecules and the purification process (e.g., by tweaking the molecular descriptors and observing the effect on various performance indicators). These insights can help guide molecular design in the future, by identifying key molecular characteristics that affect purification effectiveness.

To avoid the time and cost of having to perform and collect a very large amount of labeled historical data, interpretable machine learning models may be used as model(s) 108. For example, training server 104 may train one of model(s) 108 on hundreds of features, after which training server 104 (or a human reviewer) may analyze the trained model (e.g., weights assigned to each feature) to determine the most predictive features (e.g., about 10 features, or about 50 features, etc.). Thereafter, that particular model 108, or a new version of that model 108 that has been trained using only the most predictive features, may be used with a much smaller feature set. Identifying highly predictive features may also be useful for other purposes, such as providing new scientific insights that may give rise to new hypotheses, which could in turn lead to bioprocess improvements.

Various techniques for determining which models are best suited for particular performance indicators (and/or process parameters), and for identifying the most predictive features for a given model or use case, are now described with reference to FIGs. 3 and 4.

Generally, well-performing models for specific performance indicators may be identified by training a number of diverse model types using real-world, historical training data from previous chromatography purification processes, and comparing the results. FIG. 3 depicts an example process 300 that may be used to this end, for a particular performance indicator of interest. At a first stage 302 of the process 300, data that is relevant to the performance indicator is selected (i.e., identified and obtained). However, historical data can often be inconsistent, e.g., with different types and/or formats of data being captured for different drug products or projects. Thus, at stage 304, it may be necessary to impute missing values, and/or take other steps to ensure a robust set of training data (e.g., normalizing, removing outliers, etc.).

At stage 306, each of the candidate models is trained on at least a portion of the historical data, with hyperparameters being optimized for each candidate model. Stage 306 may include performing k-fold validation for each model (e.g., with k = 10, where a model is trained and evaluated 10 times across different 90/10 partitions of the dataset that was selected at stage 302 and augmented at stage 304, or with k = 5, etc.). Stage 306 may include tuning the hyperparameters of each model using a Bayesian search technique. The Bayesian technique performs a Bayesian-guided search that is computationally more efficient than a grid search or a random search, yet yields similar levels of performance as a random search. Stage 306 may include a number of iterations of Bayesian search, and choosing the model hyperparameters through k-fold validation.

At stage 308, the various candidate models (with their tuned hyperparameters) are evaluated, and a best model (for the performance indicator or process parameter of interest) is chosen. Any suitable criteria may be used to select a "best" model. For example, algorithm performance metrics such as the coefficient of determination ($R^2$) and/or root mean squared error (RMSE) may be captured for each model, with an average for each being obtained based on the cross-validation process. $R^2$ may be calculated as:

$$R^2 = 1 - \frac{\sum_{i=1}^{n}(\widehat{f_i} - y_i)^2}{\sum_{i=1}^{n}(\bar{y} - y_i)^2}, \qquad \text{(Equation 1)}$$

In Equation 1, n represents the number of samples per cross-validation fold, y represents the true target output, and *f* represents the output predicted by the model. Average $R^2$ may be calculated as:

$$Avg\ R^2 \equiv \frac{1}{k}\sum_{j=1}^{k} R^2{}_j\ , \quad \text{(Equation 2)}$$

where k represents the number of cross-validation folds. RMSE may be calculated as:

$$RMSE \equiv \sqrt{\frac{1}{n}\sum_{i=1}^{n}(\hat{f}_i - y_i)^2} \quad \text{(Equation 3)}$$

Average RMSE for a model may be calculated as:

$$RMSE_{avg} = \frac{1}{k}\sum_{j=1}^{k} RMSE_j \quad \text{(Equation 4)}$$

RMSE may be a better metric than $R^2$, because RMSE indicates the model accuracy/error in the easily-understood units of the performance indicator (or process parameter) being predicted. Furthermore, the $R^2$ metric can occasionally yield extremely negative values with some cross-validation sets, which can skew the model comparison when averaged across sets. RMSE may also be preferable to mean absolute error (MAE), because the former penalizes larger errors between the predictions and the actual results.

Thereafter, at stage 310, a final model for predicting the performance indicator (or process parameter) is output/selected, e.g., based on a comparison of RMSE (and/or one or more other metrics) for the different models. The final model may be re-trained on the entire dataset. The final production model is then stored as a trained model (e.g., one of ML model(s) 108), and is ready to make predictions for new/future chromatography processes.

In one embodiment, process 300 is performed by training server 104 of FIG. 1 (possibly with human input at various stages, such as selecting performance indicators of interest, selecting models as candidates, etc.). Process 300 may repeated for each performance indicator of interest, and for any suitable number of performance indicators (e.g., 5, 10, 20, etc.), such as any of the example performance indicators discussed elsewhere herein. As final models for the different performance indicators are output at each iteration of stage 310, training server 104 may add those final models to ML models 108. Thereafter, and prior to making a prediction for a particular, hypothetical chromatography purification process in the manner discussed herein (e.g., with reference to FIG. 1), computing system 102 or training server 104 may select the appropriate final model from ML models 108. The selection may be made based on user input indicating the desired performance indicator, for example.

Generally following the process 300, a number of models have been identified as having superior performance with respect to various different performance indicators for a chromatographic purification process during manufacture of a therapeutic protein, based on RMSE and as shown in Table 1 below:

TABLE 1

| Performance Indicator | Model with lowest Average RMSE | RMSE | RMSE / (RMSE range size) | Total # of Observations |
|---|---|---|---|---|
| rCE-SDS % LC + HC | Elastic Net | 0.282 | 28% | 41 |
| rCE-SDS % LC | Elastic Net | 0.170 | 28% | 71 |
| nrCE-SDS % Main | Regression Tree | 0.399 | 17% | 160 |
| rCE-SDS % Pre-LC | Regression Tree | 0.044 | 44% | 71 |
| rCE-SDS % NGHC | Regression Tree | 0.059 | 12% | 71 |
| rCE-SDS % HC | Regression Tree | 0.272 | 25% | 71 |
| rCE-SDS % HMW | Regression Tree | 0.213 | 35% | 71 |
| rCE-SDS % Pre-LC + LC + HC | Regression Tree | 0.141 | 16% | 71 |
| Pool Conductivity (mS/cm) | Regression Tree | 0.442 | 7% | 55 |
| nrCE-SDS % Pre-Peak | Regression Tree | 0.340 | 20% | 82 |
| CEX % Basic | Regression Tree | 0.741 | 2% | 492 |
| SEC % HMW | Regression Tree | 0.247 | 10% | 811 |
| SEC % Main | Regression Tree | 0.225 | 10% | 541 |
| SEC % LMW | Regression Tree | 0.035 | 3% | 339 |

(continued)

| Performance Indicator | Model with lowest Average RMSE | RMSE | RMSE / (RMSE range size) | Total # of Observations |
|---|---|---|---|---|
| CEX % Acidic | XGBoost | 0.763 | 3% | 446 |
| cIEF % Acidic | XGBoost | 1.203 | 17% | 90 |
| CEX % Main | XGBoost | 0.845 | 2% | 543 |
| cIEF % Basic | XGBoost | 0.725 | 17% | 90 |
| cIEF % Main | XGBoost | 1.214 | 18% | 90 |
| Step Yield (%) | XGBoost | 3.003 | 5% | 879 |
| rCE-SDS % Main | XGBoost | 0.483 | 18% | 86 |
| rCE-SDS % LMW | XGBoost | 0.239 | 7% | 157 |

The results of Table 1 all pertain to historical datasets for various monoclonal antibodies. For each performance indicator, the model that yielded the lowest RMSE was interpreted to be the "best performing" model. As seen in Table 1, no one model performed best for predicting all performance indicators. Rather, a regression tree model performed best for 12 performance indicators, an xgboost model performed best for eight performance indicators, and elastic net performed best for two performance indicators. Other models (lasso, ridge, SGD, linear SVM, and PLS) evaluated with the process 300 did not perform the best for any of the performance indicators in Table 1. Regression tree and xgboost models, in particular, performed well with both large and small numbers of observations (training datasets).

Other processes may have different performance characteristics, due to larger or smaller training datasets, evaluating models based on different performance indicators (and/or based on combinations of different performance indicators), evaluating models using different metrics (other than RMSE), and so on. For example, when evaluating machine learning models for predicting yield and models for predicting SEC-HMW percentages (specifically for monoclonal antibodies) according to the $R^2$ metric, the best performing model was found to be the xgboost model. For this latter evaluation, process parameter inputs to the xgboost model included column volume, load pH, actual CEX loading, loading flow rate, elution flow rate, buffer concentration, elution pH, gradient slope, gradient length, gradient start, gradient end, pool volume, protein concentration, pool start, and/or pool end.

As noted above, it may be advantageous to learn which features are most important for a particular model so that, when the "best" model is identified/output at stage 310, only those features that are most predictive of the desired performance indicator are utilized. FIGs. 4A and 4B depict plots 400, 420 of example feature importance metrics (relative feature importance and feature correlation, respectively), both for predicting experimental yield and for predicting SE-HPLC HMW, using an eXtreme gradient boost (xgboost) model. The plots 400, 420 may be generated by visualization unit 136 and presented on a GUI via display 124, for example.

Plots such as the plots 400, 420 may allow users (e.g., scientists) to easily identify the most significant factors for predicting specific performance indicators (here, to predicting experimental yield and SE-HPLC HMW). This can also provide greater insight into how the structure of a molecule affects the purification process. For example, while it is common knowledge that hydrophobicity plays a role in increasing impurity in a CEX process, it is generally believed that the charge of the molecule would have the greatest effect. However, feature importance plots similar to plots 400 and/or 420 (or correlation heat maps, etc.) consistently and surprisingly rank hydrophobicity as a more significant indicator of higher impurities (specifically, high HMW) following a CEX process. Additionally, not all forms of hydrophobicity affect the level of impurity equally. For example, plots similar to plots 420 (or correlation heat maps, etc.) show that some forms of hydrophobicity are indicative of lower impurities (specifically, less HMW), at least relative to other hydrophobicities.

FIG. 5A is a flow diagram of an example method 500 for facilitating selection of chromatography parameters for a purification process during the manufacture of therapeutic proteins. The method 500 may be implemented, at least in part, by processing unit 120 of computing system 102 when executing the software instructions of application 130 stored in memory unit 128, or by one or more processors of server 104 (e.g., in a cloud service implementation), for example.

At block 502, one or more process parameter values associated with a hypothetical chromatography (e.g., CEX, SEC, or Protein A chromatography) process are received. The process parameter value(s) may be received via a user interface, for example, and/or by importing a file or other data, etc. As examples, and without limitation, the process parameter value(s) may include values of one or more of any of the following: buffer pH, elution buffer conductivity (mS/cm), elution buffer molarity (mM), elution buffer pH, gradient slope (mM/CV), linear velocity (cm/hr), load conductivity (mS/cm), load factor (g/Lr), load pH, stop collect (%), column volume, actual CEX loading, loading flow rate, elution flow rate, buffer concentration, gradient length, gradient start, gradient end, pool volume, protein concentration, pool start, and/or pool end. While example units are shown in the preceding list, it will be appreciated that these units are for illustrative purposes only, and that these parameters may be conveyed in any suitable units. Accordingly, it will be appreciated that the example units may be omitted from the preceding list, or any other list of process parameters herein.

At block 504, one or more molecular descriptors that are descriptive of the therapeutic protein are received. The molecular descriptor(s) may be received via a user interface, for example, and/or by importing a file or other data (e.g., from MOE application 139), etc. In some embodiments, method 500 also includes determining one or more molecular descriptors based on sequence information associated with the therapeutic protein (e.g., amino acid sequence information entered into MOE software), and/or based on an experimental measurement of a physical characteristic of the therapeutic protein (e.g., a measurement result entered into MOE software). In some embodiments, at least one molecular descriptor is a function of pH of the environment surrounding the molecule (e.g., a mathematical function that varies when a pH is known/specified). As examples, and without limitation, the molecular descriptor(s) may include any one or more of the following: pH, HI, pro_Fv_net_charge, U, asa_hyd, viscosity, hyd_idx, pro_helicity, apol, asa_hph, pro_net_charge, hyd_idx_cdr, pro_henry, b_1rotR, volume, amphipathicity, hyd_strength, pro_hyd_moment, b_rotR, mobility, ASPmax, hyd_strength_cdr, pro_mass, density, helicity, BSA, Packing Score, pro_mobility, ens_dipole, henry, BSA_HC, pro_affinity, pro_pl_3D, mass, net_charge, BSA_LC_HC, pro_app_charge, pro_pl_seq, pl_seq, app_charge, contact energy, pro_asa_hph, pro_r_gyr, pl_3D, dipole_moment, DRT, pro_asa_hyd, pro_r_solv, coeff_fric, hyd_moment, E bond, pro_asa_vdw, pro_sed_const, coeff_diff, zeta, E ele, pro_cdr_net_charge, pro_stability, r_gyr, zdipole, E sol, pro_coeff_diff, pro_volume, r_solv, zquadrupole, E vdw, pro_coeff_fric, pro_zdipole, sed_const, Eint_VL_VH, pro_dipole_moment, pro_zeta, eccen, GBNI, pro_eccen, pro_zquadrupole, and/or asa_vdw.

At block 506, a performance indicator for the hypothetical chromatography process is predicted, at least by analyzing the process parameter(s) received at block 502, and the molecular descriptor(s) received at block 504, using a machine learning model. The machine learning model may be either a regression tree model, an eXtreme gradient boost (xgboost) model, or an elastic net model. As examples, and without limitation, the predicted performance indicator may be one of the following: Step Yield, CEX % Acidic, CEX % Main, CEX % Basic, SEC % HMW, SEC % Main, SEC % low molecular weight (LMW), capillary electrophoresis sodium dodecyl sulfate with reduced sample preparation (rCE-SDS) % Main, rCE-SDS % LMW, rCE-SDS % light chain (LC) + heavy chain (HC), capillary electrophoresis sodium dodecyl sulfate without reduced sample preparation (nrCE-SDS) % Main, rCE-SDS % Pre-LC, rCE-SDS % LC, rCE-SDS % non-glycosylated heavy chain (NGHC), rCE-SDS % HC, rCE-SDS % high molecular weight (HMW), rCE-SDS % Pre-LC+LC+HC, pool conductivity (mS/cm), capillary isoelectric focusing (cIEF) % Acidic, cIEF % Basic, cIEF % Main, nrCE-SDS % Pre-Peak, HCP, and/or SE-HPLC HMW.

In some embodiments, block 506 includes using a regression tree model to predict nrCE-SDS % LC + HC, rCE-SDS % Pre-LC, CEX % Basic, SEC % HMW, SEC % Main, SEC % LMW, rCE-SDS % HC, rCE-SDS % HMW, rCE-SDS % Pre-LC+LC_HC, pool conductivity, or nrCE-SDS % Pre-Peak. In other embodiments, block 506 includes using an eXtreme gradient boost model to predict CEX % Acidic, CEX % Main, step yield, rCE-SDS % Main, rCE-SDS % LMW, cIEF % Acidic, cIEF % Basic, or cIEF % Main. Alternatively, block 506 may include using an eXtreme gradient boost model to predict SEC % HMW or yield. In still other embodiments, block 506 includes using an elastic net model to predict rCE-SDS % LC + HC, or to predict rCE-SDS % LC.

At block 508, the performance indicator predicted at block 506, and/or an indication of whether the predicted performance indicator satisfies one or more acceptability criteria (e.g., exceeds, or is below, some threshold value), is caused to be presented to a user via a user interface (e.g., a GUI generated or populated by visualization unit 136 and presented on display 124 of FIG. 1), to facilitate the selection (e.g., manual selection by a user) of chromatography parameters for a real-world purification process during the manufacture of the therapeutic protein.

In some embodiments, the method 500 includes one or more additional blocks not shown in FIG. 5A. For example, method 500 may include two additional blocks that both occur prior to block 502: a first additional block in which data indicative of a performance indicator of interest is received from a user via a user interface (e.g., a GUI generated or populated by visualization unit 136 and presented on display 124), and a second additional block in which the machine learning model (later used at block 506) is selected from among multiple machine learning models (e.g., ML models 108) that were trained to predict different performance indicators.

As another example, method 500 may include four additional blocks, similar to blocks 506 and 508 (or 502 through 508), to occur for a second performance indicator of interest, using a second machine learning model. For example, the first and second machine learning models may be xgboost models that were trained for a different purpose, with one predicting experimental yield percentage and the other predicting SEC HMW percentage.

As yet another example, method 500 may include two additional blocks that both occur after block 506: a first additional block in which one or more process parameter values are selected for a (real-world) chromatography process for the therapeutic protein based on the performance indicator and/or the indication presented at block 508, and a second additional block in which the chromatography process is performed for the therapeutic protein according to the one or more selected process parameter values.

FIG. 5B is a flow diagram of another example method 520 for facilitating selection of chromatography parameters for a purification process during the manufacture of therapeutic proteins. The method 520 may be implemented, at least in part, by processing unit 120 of computing system 102 when executing the software instructions of application 130 stored in memory unit 128, or by one or more processors of server 104 (e.g., in a cloud service implementation), for example.

At block 522, one or more performance indicators associated with a hypothetical chromatography (e.g., CEX, SEC, or Protein A chromatography) process are received. The performance indicator(s) may be received via a user interface, for example, and/or by importing a file or other data, etc. As examples, and without limitation, the performance indicator(s) may include values of one or more of any of the following: Step Yield, CEX % Acidic, CEX % Main, CEX % Basic, SEC % HMW, SEC % Main, SEC % low molecular weight (LMW), capillary electrophoresis sodium dodecyl sulfate with reduced sample preparation (rCE-SDS) % Main, rCE-SDS % LMW, rCE-SDS % light chain (LC) + heavy chain (HC), capillary electrophoresis sodium dodecyl sulfate without reduced sample preparation (nrCE-SDS) % Main, rCE-SDS % Pre-LC, rCE-SDS % LC, rCE-SDS % non-glycosylated heavy chain (NGHC), rCE-SDS % HC, rCE-SDS % high molecular weight (HMW), rCE-SDS % Pre-LC+LC+HC, pool conductivity (mS/cm), capillary isoelectric focusing (cIEF) % Acidic, cIEF % Basic, cIEF % Main, nrCE-SDS % Pre-Peak, HCP, and/or SE-HPLC HMW.

At block 524, one or more molecular descriptors that are descriptive of the therapeutic protein are received. The molecular descriptor(s) may be received via a user interface, for example, and/or by importing a file or other data (e.g., from MOE application 139), etc. In some embodiments, method 520 also includes determining one or more molecular descriptors based on sequence information associated with the therapeutic protein (e.g., amino acid sequence information entered into MOE software), and/or based on an experimental measurement of a physical characteristic of the therapeutic protein (e.g., a measurement result entered into MOE software). In some embodiments, at least one molecular descriptor is a function of pH of the environment surrounding the molecule (e.g., a mathematical function that varies when a pH is known/specified). As examples, and without limitation, the molecular descriptor(s) may include any one or more of the following: pH, HI, pro_Fv_net_charge, U, asa_hyd, viscosity, hyd_idx, pro_helicity, apol, asa_hph, pro_net_charge, hyd_idx_cdr, pro_henry, b_1rotR, volume, amphipathicity, hyd_strength, pro_hyd_moment, b_rotR, mobility, ASPmax, hyd_strength_cdr, pro_mass, density, helicity, BSA, Packing Score, pro_mobility, ens_dipole, henry, BSA_HC, pro_affinity, pro_pl_3D, mass, net_charge, BSA_LC_HC, pro_app_charge, pro_pl_seq, pl_seq, app_charge, contact energy, pro_asa_hph, pro_r_gyr, pl_3D, dipole_moment, DRT, pro_asa_hyd, pro_r_solv, coeff_fric, hyd_moment, E bond, pro_asa_vdw, pro_sed_const, coeff_diff, zeta, E ele, pro_cdr_net_charge, pro_stability, r_gyr, zdipole, E sol, pro_coeff_diff, pro_volume, r_solv, zquadrupole, E vdw, pro_coeff_fric, pro_zdipole, sed_const, Eint_VL_VH, pro_dipole_moment, pro_zeta, eccen, GBNI, pro_eccen, pro_zquadrupole, and/or asa_vdw.

At block 526, a process parameter value for the hypothetical chromatography process is predicted, at least by analyzing the performance indicator(s) received at block 522, and the molecular descriptor(s) received at block 524, using a machine learning model. The machine learning model may be either a regression tree model, an eXtreme gradient boost (xgboost) model, or an elastic net model. As examples, and without limitation, the process parameter for which a value is predicted may be one of the following: buffer pH, elution buffer conductivity (mS/cm), elution buffer molarity (mM), elution buffer pH, gradient slope (mM/CV), linear velocity (cm/hr), load conductivity (mS/cm), load factor (g/Lr), load pH, stop collect (%), column volume, actual CEX loading, loading flow rate, elution flow rate, buffer concentration, gradient length, gradient start, gradient end, pool volume, protein concentration, pool start, and/or pool end. While example units are shown in the preceding list, it will be appreciated that these units are for illustrative purposes only, and that these parameters may be conveyed in any suitable units. Accordingly, it will be appreciated that the example units may be omitted from the preceding list, or any other list of process parameters herein.

In some embodiments, block 526 includes using a regression tree model to predict the process parameter value based on the molecular descriptor(s) and one or more of nrCE-SDS % LC + HC, rCE-SDS % Pre-LC, CEX % Basic, SEC % HMW, SEC % Main, SEC % LMW, rCE-SDS % HC, rCE-SDS % HMW, rCE-SDS % Pre-LC+LC_HC, pool conductivity, and/or nrCE-SDS % Pre-Peak. In other embodiments, block 526 includes using an eXtreme gradient boost model to predict the process parameter value based on the molecular descriptor(s) and one or more of CEX % Acidic, CEX % Main, step yield, rCE-SDS % Main, rCE-SDS % LMW, cIEF % Acidic, cIEF % Basic, and/or cIEF % Main. Alternatively, block 506 may include using an eXtreme gradient boost model to predict the process parameter value based on the molecular descriptor(s) and one or both of SEC % HMW and yield. In still other embodiments, block 506 includes using an elastic net model to predict the process parameter value based on the molecular descriptor(s) and rCE-SDS % LC + HC, or based on the molecular descriptor(s) and rCE-SDS % LC.

At block 528, the process parameter value predicted at block 526, and/or a predicted accuracy range of the predicted process parameter value, is caused to be presented to a user via a user interface (e.g., a GUI generated or populated by visualization unit 136 and presented on display 124 of FIG. 1), to facilitate the selection (e.g., manual selection by a user) of chromatography parameters for a real-world purification process during the manufacture of the therapeutic protein.

In some embodiments, the method 520 includes one or more additional blocks not shown in FIG. 5B. For example, method 520 may include two additional blocks that both occur prior to block 522: a first additional block in which data indicative of a process parameter of interest is received from a user via a user interface (e.g., a GUI generated or populated by visualization unit 136 and presented on display 124), and a second additional block in which the machine learning model (later used at block 526) is selected from among multiple machine learning models (e.g., ML models 108) that were trained to predict values of different process parameters.

As another example, method 520 may include four additional blocks, similar to blocks 526 and 528 (or 522 through 528), to occur for a second process parameter of interest, using a second machine learning model. For example, the first and second machine learning models may be xgboost models that were trained for a different purpose, with one predicting buffer pH and the other predicting load factor.

As yet another example, method 520 may include two additional blocks that both occur after block 526: a first additional block in which one or more process parameter values are selected for a (real-world) chromatography process for the therapeutic protein based on the information presented at block 528, and a second additional block in which the chromatography process is performed for the therapeutic protein according to the one or more selected process parameter values.

Although the systems, methods, devices, and components thereof, have been described in terms of exemplary embodiments, they are not limited thereto. The detailed description is to be construed as exemplary only and does not describe every possible embodiment of the invention because describing every possible embodiment would be impractical, if not impossible. Numerous alternative embodiments could be implemented, using either current technology or technology developed after the filing date of this patent that would still fall within the scope of the claims defining the invention.

Those skilled in the art will recognize that a wide variety of modifications, alterations, and combinations can be made with respect to the above described embodiments without departing from the scope of the invention.

## Claims

1. A method for facilitating selection of chromatography parameters for a purification process during manufacture of a therapeutic protein, the method comprising:

    receiving, by one or more processors of a computing system, one or more process parameter values associated with a hypothetical chromatography process, wherein the one or more process parameter values include one or more of: buffer pH; elution buffer pH; elution buffer conductivity; elution buffer molarity; gradient slope; linear velocity; load conductivity; load factor; load pH; or stop collect;
    receiving, by the one or more processors, one or more molecular descriptors descriptive of the therapeutic protein;
    selecting a machine learning model from a group consisting of (i) a regression tree model, (ii) an eXtreme gradient boost model, and (iii) an elastic net model, wherein each model has been trained with a corresponding set of training data;
    predicting, by the one or more processors, a performance indicator for the hypothetical chromatography process, as an output of the machine learning model, at least by applying the one or more process parameter values and the one or more molecular descriptors as inputs to the machine learning model, wherein the regression tree model is selected for predicting a first set of one or more performance indicators, the eXtreme gradient boost model is selected for predicting a different, second set of one or more performance indicators and the elastic net model is selected for a different, third set of one or performance indicators, wherein:

        the first set of one or more performance indicators includes:

            capillary electrophoresis sodium dodecyl sulfate without reduced sample preparation percent light chain plus heavy chain (nrCE-SDS % LC + HC);
            capillary electrophoresis sodium dodecyl sulfate with reduced sample preparation percent pre-light chain (rCE-SDS % Pre-LC);
            cation-exchange chromatography percent Basic (CEX % Basic);
            size-exclusion chromatography percent high molecular weight (SEC % HMW);
            size-exclusion chromatography percent Main (SEC % Main);
            size-exclusion chromatography percent low molecular weight (SEC % LMW);
            capillary electrophoresis sodium dodecyl sulfate with reduced sample preparation percent heavy chain (rCE-SDS % HC);
            capillary electrophoresis sodium dodecyl sulfate with reduced sample preparation percent high molecular weight (rCE-SDS % HMW);
            capillary electrophoresis sodium dodecyl sulfate with reduced sample preparation percent pre-light chain plus light chain_heavy chain (rCE-SDS % Pre-LC+LC_HC);

pool conductivity; and/or
capillary electrophoresis sodium dodecyl sulfate without reduced sample preparation percent Pre_Peak (nrCE-SDS % Pre-Peak),

the second set of one or more performance indicators includes:

cation-exchange chromatography percent Acidic (CEX % Acidic);
cation-exchange chromatography percent Main (CEX % Main);
step yield;
capillary electrophoresis sodium dodecyl sulfate with reduced sample preparation percent (rCE-SDS % Main);
capillary electrophoresis sodium dodecyl sulfate with reduced sample preparation percent (rCE-SDS % LMW);
capillary isoelectric focusing percent acidic (cIEF % Acidic);
capillary isoelectric focusing percent basic (cIEF % Basic); and/or
capillary isoelectric focusing percent main (cIEF % Main), and

the third set of one or more performance indicators includes:

capillary electrophoresis sodium dodecyl sulfate with reduced sample preparation percent light chain plus heavy chain (rCE-SDS % LC + HC); and/or
capillary electrophoresis sodium dodecyl sulfate with reduced sample preparation percent light chain (rCE-SDS % LC); and

causing, by the one or more processors, one or both of (i) the predicted performance indicator, and (ii) an indication of whether the predicted performance indicator satisfies one or more acceptability criteria, to be presented to a user via a user interface.

**2.** A method for facilitating selection of chromatography parameters for a purification process during manufacture of a therapeutic protein, the method comprising:

receiving, by one or more processors of a computing system, one or more performance indicators associated with a hypothetical chromatography process, wherein the one or more performance indicators may be chosen from a first set of performance indicators, a second set of performance indicators, or a third set of performance indicators, and wherein:

the first set of performance indicators includes:

capillary electrophoresis sodium dodecyl sulfate without reduced sample preparation percent light chain plus heavy chain (nrCE-SDS % LC + HC);
capillary electrophoresis sodium dodecyl sulfate with reduced sample preparation percent pre-light chain (rCE-SDS % Pre-LC);
cation-exchange chromatography percent Basic (CEX % Basic);
size-exclusion chromatography percent high molecular weight (SEC % HMW);
size-exclusion chromatography percent Main (SEC % Main);
size-exclusion chromatography percent low molecular weight (SEC % LMW);
capillary electrophoresis sodium dodecyl sulfate with reduced sample preparation percent heavy chain (rCE-SDS % HC);
capillary electrophoresis sodium dodecyl sulfate with reduced sample preparation percent high molecular weight (rCE-SDS % HMW);
capillary electrophoresis sodium dodecyl sulfate with reduced sample preparation percent pre-light chain plus light chain_heavy chain (rCE-SDS % Pre-LC+LC_HC);
pool conductivity; and/or
capillary electrophoresis sodium dodecyl sulfate without reduced sample preparation percent Pre_Peak (nrCE-SDS % Pre-Peak),

the second set of performance indicators includes:

cation-exchange chromatography percent Acidic (CEX % Acidic);
cation-exchange chromatography percent Main (CEX % Main);
step yield;
capillary electrophoresis sodium dodecyl sulfate with reduced sample preparation percent (rCE-SDS % Main);
capillary electrophoresis sodium dodecyl sulfate with reduced sample preparation percent (rCE-SDS % LMW);
capillary isoelectric focusing percent acidic (cIEF % Acidic);
capillary isoelectric focusing percent basic (cIEF % Basic); and/or
capillary isoelectric focusing percent main (cIEF % Main), and

the third set of performance indicators includes:

capillary electrophoresis sodium dodecyl sulfate with reduced sample preparation percent light chain plus heavy chain (rCE-SDS % LC + HC); and/or
capillary electrophoresis sodium dodecyl sulfate with reduced sample preparation percent light chain (rCE-SDS % LC);

receiving, by the one or more processors, one or more molecular descriptors descriptive of the therapeutic protein;
selecting a machine learning model from a group consisting of (i) a regression tree model, (ii) an eXtreme gradient boost model, and (iii) an elastic net model, wherein each model has been trained with a corresponding set of training data;
predicting, by the one or more processors, a process parameter value for a process parameter associated with the hypothetical chromatography process, as an output of the machine learning model, at least by applying the one or more performance indicators and the one or more molecular descriptors as inputs to the machine learning model, wherein the process parameter value includes: buffer pH; elution buffer pH; elution buffer conductivity; elution buffer molarity; gradient slope; linear velocity; load conductivity; load factor; load pH; or stop collect, wherein the one or more performance indicators indicate an aspect of performance of the hypothetical chromatography process, wherein the one or more process parameters include parameters relating to conditions or characteristics of the hypothetical chromatography process, and wherein the regression tree model is selected for predicting the process parameter value when the one or more performance indicators are included in the first set of performance indicators, the eXtreme gradient boost model is selected for predicting the process parameter when the one or more performance indicators are included in the second set of performance indicators and the elastic net model is selected for predicting the process parameter when the one or more performance indicators are included in the third set of performance indicators; and
causing, by the one or more processors, one or both of (i) the predicted process parameter value, and (ii) a predicted accuracy range of the predicted process parameter value, to be presented to a user via a user interface.

**3.** The method of claim 1 or 2, wherein the hypothetical chromatography process is a process selected from a group consisting of:

a hypothetical cation-exchange chromatography (CEX) process;
a hypothetical size-exclusion chromatography (SEC) process; and
a Protein A chromatography process.

**4.** The method of any one of claims 1 through 3, further comprising:
determining, by the one or more processors, at least one of the one or more molecular descriptors based on sequence information associated with the therapeutic protein.

**5.** The method of any one of claims 1 through 4, further comprising:
determining, by the one or more processors, at least one of the one or more molecular descriptors based on an experimental measurement of a physical characteristic of the therapeutic protein.

**6.** The method of any one of claims 1 through 5, wherein at least one of the one or more molecular descriptors is a function of pH level.

**7.** The method of any one of claims 1 or 3 through 6, wherein:

the performance indicator includes SEC % HMW; and
the method further comprises:

predicting, by the one or more processors, a yield for the hypothetical chromatography process at least by analyzing process parameters and molecular descriptors using an additional machine learning model, wherein the additional machine learning model is another eXtreme gradient boost model; and
causing, by the one or more processors, one or both of (i) the predicted yield, and (ii) an indication of whether the predicted yield satisfies one or more additional acceptability criteria, to be presented to the user via the user interface.

**8.** The method of claim 1, further comprising receiving data indicative of a performance indicator of interest from a user via a user interface.

**9.** The method of claim 1, further comprising:

selecting one or more process parameter values for a chromatography process for the therapeutic protein based on the presented performance indicator and/or the presented indication; and
performing the chromatography process for the therapeutic protein according to the selected process parameter values.

**10.** The method of claim 2, further comprising:

selecting one or more process parameter values for a chromatography process for the therapeutic protein based on the presented predicted process parameter value, and/or the predicted accuracy range; and
performing the chromatography process for the therapeutic protein according to the selected process parameter values.

**11.** One or more non-transitory, computer-readable media storing instructions that, when executed by one or more processors of a computing system, cause the computing system to perform the method of any one of claims 1 through 8.

**12.** A computing system comprising:

one or more processors; and
one or more non-transitory, computer-readable media storing instructions that, when executed by the one or more processors, cause the computing system to perform the method of any one of claims 1 through 8.

## Patentansprüche

**1.** Verfahren zur Erleichterung der Auswahl von Chromatographieparametern für ein Reinigungsverfahren während der Herstellung eines therapeutischen Proteins, wobei das Verfahren Folgendes umfasst:

Empfangen, durch einen oder mehrere Prozessoren eines Computersystems, eines oder mehrerer mit einem hypothetischen Chromatographieverfahren assoziierter Verfahrensparameterwerte, wobei der eine oder die mehreren Verfahrensparameterwerte eines oder mehrere der Folgendem beinhalten: Puffer-pH, Elutionspuffer-pH, Elutionspufferleitfähigkeit, Elutionspuffermolarität, Gradientensteigung, Lineargeschwindigkeit, Lastleitfähigkeit, Lastfaktor, Last-pH oder Sammeln-Stopp,
Empfangen, durch den einen oder die mehreren Prozessoren, eines oder mehrerer molekularer Deskriptoren, die das therapeutische Protein beschreiben,
Auswählen eines Maschinenlernmodells aus einer Gruppe, bestehend aus (i) einem Regressionsbaum-Modell, (ii) einem eXtreme Gradient Boost-Modell und (iii) einem elastisches-Netz-Modell, wobei jedes Modell mit einem entsprechenden Satz von Trainingsdaten trainiert worden ist,
Vorhersagen, durch den einen oder die mehreren Prozessoren, eines Leistungsindikators für das hypothetische Chromatographieverfahren als Ausgabe des Maschinenlernmodells, zumindest durch Anwenden des einen oder der mehreren Verfahrensparameterwerte und des einen oder der mehreren molekularen Deskriptoren als Eingaben in das Maschinenlernmodell, wobei das Regressionsbaum-Modell zum Vorhersagen eines ersten Satzes von einem oder mehreren Leistungsindikatoren ausgewählt wird, das extreme Gradient Boost-Modell zum Vorhersagen eines anderen, zweiten Satzes von einem oder mehreren Leistungsindikatoren ausgewählt wird und das elastisches-Netz-Modell für einen anderen, dritten Satz von einem oder mehreren Leistungsindikatoren ausgewählt wird, wobei:
der erste Satz von einem oder mehreren Leistungsindikatoren Folgendes beinhaltet:

Kapillarelektrophorese Natriumdodecylsulfat ohne Herstellung reduzierter Proben prozentualer Anteil leichte Kette plus schwere Kette (nrCE-SDS % LC + HC),
Kapillarelektrophorese Natriumdodecylsulfat mit Herstellung reduzierter Proben prozentualer Anteil Präleichte Kette (rCE-SDS % Pre-LC),
Kationenaustauschchromatographie prozentualer Anteil Basische (CEX % Basic),
Größenausschlusschromatographie prozentualer Anteil Hochmolekulare (SEC % HMW),
Größenausschlusschromatographie prozentualer Anteil Hauptkomponente (SEC % Main),
Größenausschlusschromatographie prozentualer Anteil Niedermolekulare (SEC % LMW),
Kapillarelektrophorese Natriumdodecylsulfat mit Herstellung reduzierter Proben prozentualer Anteil schwere Kette (rCE-SDS % HC),
Kapillarelektrophorese Natriumdodecylsulfat mit Herstellung reduzierter Proben prozentualer Anteil Hochmolekulare (rCE-SDS % HMW),
Kapillarelektrophorese Natriumdodecylsulfat mit Herstellung reduzierter Proben prozentualer Anteil Präleichte Kette plus leichte Kette_schwere Kette (rCE-SDS % Pre-LC+LC_HC),
Pool-Leitfähigkeit und/oder
Kapillarelektrophorese Natriumdodecylsulfat ohne Herstellung reduzierter Proben prozentualer Anteil Vor_Peak (nrCE-SDS % Pre-Peak),
der zweite Satz von einem oder mehreren Leistungsindikatoren Folgendes beinhaltet:

Kationenaustauschchromatographie prozentualer Anteil Saure (CEX % Acidic),
Kationenaustauschchromatographie prozentualer Anteil Hauptkomponente (CEX % Main),
Schrittausbeute,
Kapillarelektrophorese Natriumdodecylsulfat mit Herstellung reduzierter Proben prozentualer Anteil (rCE-SDS % Main),
Kapillarelektrophorese Natriumdodecylsulfat mit Herstellung reduzierter Proben prozentualer Anteil (rCE-SDS % LMW),
Kapillar-isoelektrische Fokussierung prozentualer Anteil Saure (cIEF % Acidic),
Kapillar-isoelektrische Fokussierung prozentualer Anteil Basische (cIEF % Basic) und/oder
Kapillar-isoelektrische Fokussierung prozentualer Anteil Hauptkomponente (cIEF % Main), und
der dritte Satz von einem oder mehreren Leistungsindikatoren Folgendes beinhaltet:

Kapillarelektrophorese Natriumdodecylsulfat mit Herstellung reduzierter Proben prozentualer Anteil leichte Kette plus schwere Kette (rCE-SDS % LC + HC) und/oder
Kapillarelektrophorese Natriumdodecylsulfat mit Herstellung reduzierter Proben prozentualer Anteil leichte Kette (rCE-SDS % LC), und
Veranlassen, durch den einen oder die mehreren Prozessoren, dass eines oder beide aus (i) dem vorhergesagten Leistungsindikator und (ii) einem Hinweis, ob der vorhergesagte Leistungsindikator ein oder mehrere Akzeptanzkriterien erfüllt, einem Benutzer über eine Benutzerschnittstelle dargestellt wird bzw. werden.

**2.** Verfahren zur Erleichterung der Auswahl von Chromatographieparametern für ein Reinigungsverfahren während der Herstellung eines therapeutischen Proteins, wobei das Verfahren Folgendes umfasst:
Empfangen, durch einen oder mehrere Prozessoren eines Computersystems, eines oder mehrerer mit einem hypothetischen Chromatographieverfahren assoziierter Leistungsindikatoren, wobei der eine oder die mehreren Leistungsindikatoren aus einem ersten Satz von Leistungsindikatoren, einem zweiten Satz von Leistungsindikatoren oder einem dritten Satz von Leistungsindikatoren ausgewählt werden können und wobei: der erste Satz von einem oder mehreren Leistungsindikatoren Folgendes beinhaltet:

Kapillarelektrophorese Natriumdodecylsulfat ohne Herstellung reduzierter Proben prozentualer Anteil leichte Kette plus schwere Kette (nrCE-SDS % LC + HC),
Kapillarelektrophorese Natriumdodecylsulfat mit Herstellung reduzierter Proben prozentualer Anteil Präleichte Kette (rCE-SDS % Pre-LC),
Kationenaustauschchromatographie prozentualer Anteil Basische (CEX % Basic),

Größenausschlusschromatographie prozentualer Anteil Hochmolekulare (SEC % HMW),
Größenausschlusschromatographie prozentualer Anteil Hauptkomponente (SEC % Main),
Größenausschlusschromatographie prozentualer Anteil Niedermolekulare (SEC % LMW),
Kapillarelektrophorese Natriumdodecylsulfat mit Herstellung reduzierter Proben prozentualer Anteil schwere Kette (rCE-SDS % HC),
Kapillarelektrophorese Natriumdodecylsulfat mit Herstellung reduzierter Proben prozentualer Anteil Hochmolekulare (rCE-SDS % HMW),
Kapillarelektrophorese Natriumdodecylsulfat mit Herstellung reduzierter Proben prozentualer Anteil Präleichte Kette plus leichte Kette_schwere Kette (rCE-SDS % Pre-LC+LC_HC),
Pool-Leitfähigkeit und/oder
Kapillarelektrophorese Natriumdodecylsulfat ohne Herstellung reduzierter Proben prozentualer Anteil Vor_Peak (nrCE-SDS % Pre-Peak),
der zweite Satz von Leistungsindikatoren Folgendes beinhaltet:

Kationenaustauschchromatographie prozentualer Anteil Saure (CEX % Acidic),
Kationenaustauschchromatographie prozentualer Anteil Hauptkomponente (CEX % Main),
Schrittausbeute,
Kapillarelektrophorese Natriumdodecylsulfat mit Herstellung reduzierter Proben prozentualer Anteil (rCE-SDS % Main),
Kapillarelektrophorese Natriumdodecylsulfat mit Herstellung reduzierter Proben prozentualer Anteil (rCE-SDS % LMW),
Kapillar-isoelektrische Fokussierung prozentualer Anteil Saure (cIEF % Acidic),
Kapillar-isoelektrische Fokussierung prozentualer Anteil Basische (cIEF % Basic) und/oder
Kapillar-isoelektrische Fokussierung prozentualer Anteil Hauptkomponente (cIEF % Main), und
der dritte Satz von Leistungsindikatoren Folgendes beinhaltet:

Kapillarelektrophorese Natriumdodecylsulfat mit Herstellung reduzierter Proben prozentualer Anteil leichte Kette plus schwere Kette (rCE-SDS % LC + HC) und/oder
Kapillarelektrophorese-Natriumdodecylsulfat mit Herstellung reduzierter Proben prozentualer Anteil leichte Kette (rCE-SDS % LC),
Empfangen, durch den einen oder die mehreren Prozessoren, eines oder mehrerer molekularer Deskriptoren, die das therapeutische Protein beschreiben,
Auswählen eines Maschinenlernmodells aus einer Gruppe, bestehend aus (i) einem Regressionsbaum-Modell, (ii) einem extreme Gradient Boost-Modell und (iii) einem elastisches-Netz-Modell, wobei jedes Modell mit einem entsprechenden Satz von Trainingsdaten trainiert worden ist,
Vorhersagen, durch den einen oder die mehreren Prozessoren, eines Verfahrensparameterwerts für einen mit dem hypothetischen Chromatographieverfahren assoziierten Verfahrensparameter als Ausgabe des Maschinenlernmodells, zumindest durch Anwenden des einen oder der mehreren Leistungsindikatoren und des einen oder der mehreren molekularen Deskriptoren als Eingaben in das Maschinenlernmodell, wobei der Verfahrensparameterwert Folgendes beinhaltet: Puffer-pH, Elutionspuffer-pH, Elutionspufferleitfähigkeit, Elutionspuffermolarität, Gradientensteigung, Lineargeschwindigkeit, Lastleitfähigkeit, Lastfaktor, Last-pH oder Sammel-Stopp, wobei der eine oder die mehreren Leistungsindikatoren einen Aspekt der Leistung des hypothetischen Chromatographieverfahrens angeben, wobei der eine oder die mehreren Verfahrensparameter Parameter in Bezug auf Bedingungen oder Merkmale des hypothetischen Chromatographieverfahrens beinhalten und wobei das Regressionsbaum-Modell zum Vorhersagen des Verfahrensparameterwerts ausgewählt wird, wenn der eine oder die mehreren Leistungsindikatoren in dem ersten Satz von Leistungsindikatoren enthalten ist bzw. sind, das extreme Gradient Boost-Modell zum Vorhersagen des Verfahrensparameters ausgewählt wird, wenn der eine oder die mehreren Leistungsindikatoren in dem zweiten Satz von Leistungsindikatoren enthalten ist bzw. sind, und das elastisches-Netz-Modell zum Vorhersagen des Verfahrensparameters ausgewählt wird, wenn der eine oder die mehreren Leistungsindikatoren in dem dritten Satz von Leistungsindikatoren enthalten sind, und
Veranlassen, durch den einen oder die mehreren Prozessoren, dass eines oder beide aus (i) dem vorhergesagten Leistungsindikator und (ii) einem vorhergesagten Genauigkeitsbereich des vorhergesagten Verfahrensparameterwerts einem Benutzer über eine Benutzerschnittstelle dargestellt wird bzw. werden.

**3.** Verfahren nach Anspruch 1 oder 2, wobei das hypothetische Chromatographieverfahren ein Verfahren ist, das aus einer aus den Folgenden bestehenden Gruppe ausgewählt ist:

einem hypothetischen Kationenaustauschchromatographie(CEX)-Verfahren,
einem hypothetischen Größenausschlusschromatographie(SEC)-Verfahren und einem Protein-A-Chromatographie-Verfahren.

**4.** Verfahren nach einem der Ansprüche 1 bis 3, das ferner Folgendes umfasst:
Bestimmen, durch den einen oder die mehreren Prozessoren, von mindestens einem aus dem einen oder den mehreren molekularen Deskriptoren auf der Basis von mit dem therapeutischen Protein assoziierten Sequenzinformationen.

**5.** Verfahren nach einem der Ansprüche 1 bis 4, das ferner Folgendes umfasst:
Bestimmen, durch den einen oder die mehreren Prozessoren, von mindestens einem aus dem einen oder den mehreren molekularen Deskriptoren auf der Basis einer experimentellen Messung eines physikalischen Merkmals des therapeutischen Proteins.

**6.** Verfahren nach einem der Ansprüche 1 bis 5, wobei mindestens einer aus dem einen oder den mehreren molekularen Deskriptoren eine Funktion des pH-Niveaus ist.

**7.** Verfahren nach einem der Ansprüche 1 oder 3 bis 6, wobei:

der Leistungsindikator SEC % HMW einschließt und
das Verfahren ferner Folgendes umfasst:

Vorhersagen, durch den einen oder die mehreren Prozessoren, einer Ausbeute für das hypothetische Chromatographieverfahren zumindest durch Analysieren von Verfahrensparametern und molekularen Deskriptoren unter Verwendung eines zusätzlichen Maschinenlernmodells, wobei das zusätzliche Maschinenlernmodell ein weiteres extreme Gradient Boost-Modell ist, und
Veranlassen, durch den einen oder die mehreren Prozessoren, dass eines oder beide aus (i) der vorhergesagten Ausbeute und (ii) einem Hinweis, ob die vorhergesagte Ausbeute ein oder mehrere zusätzliche Akzeptanzkriterien erfüllt, dem Benutzer über die Benutzeroberfläche dargestellt werden.

**8.** Verfahren nach Anspruch 1, das ferner das Empfangen von Daten, die einen Leistungsindikator von Interesse angeben, von einem Benutzer über eine Benutzerschnittstelle umfasst.

**9.** Verfahren nach Anspruch 1, das ferner Folgendes umfasst:

Auswählen eines oder mehrerer Verfahrensparameterwerte für ein Chromatographieverfahren für das therapeutische Protein auf der Basis des dargestellten Leistungsindikators und/oder des dargestellten Hinweises und Durchführen des Chromatographieverfahrens für das therapeutische Protein gemäß den ausgewählten Verfahrensparameterwerten.

**10.** Verfahren nach Anspruch 2, das ferner Folgendes umfasst:

Auswählen eines oder mehrerer Verfahrensparameterwerte für ein Chromatographieverfahren für das therapeutische Protein auf der Basis des dargestellten vorhergesagten Verfahrensparameterwerts und/oder des vorhergesagten Genauigkeitsbereichs und
Durchführen des Chromatographieverfahrens für das therapeutische Protein gemäß den ausgewählten Verfahrensparameterwerten.

**11.** Nicht-flüchtiges computerlesbares Medium bzw. nicht-flüchtige computerlesbare Medien, das bzw. die Anweisungen speichert bzw. speichern, die, wenn sie von einem oder mehreren Prozessoren eines Computersystems ausgeführt werden, das Computersystem veranlassen, das Verfahren nach einem der Ansprüche 1 bis 8 auszuführen.

**12.** Computersystem, umfassend:

einen oder mehrere Prozessoren und
ein nicht-flüchtiges computerlesbares Medium bzw. nicht-flüchtige computerlesbare Medien, das bzw. die Anweisungen speichert bzw. speichern, die, wenn sie durch einen oder mehrere Prozessoren eines Computersystems ausgeführt werden, das Computersystem veranlassen, das Verfahren nach einem der Ansprüche 1 bis 8 auszuführen.

## Revendications

1. Procédé de facilitation de sélection de paramètres de chromatographie pour un processus de purification durant la fabrication d'une protéine thérapeutique, le procédé comprenant :

la réception, par un ou plusieurs processeurs d'un système informatique, d'une ou plusieurs valeurs de paramètre de processus associées à un processus de chromatographie hypothétique, dans lequel l'une ou les plusieurs valeurs de paramètre de processus incluent un ou plusieurs éléments parmi : pH de tampon ; pH de tampon d'élution ; conductivité de tampon d'élution ; molarité de tampon d'élution ; pente de gradient ; vélocité linéaire ; conductivité de charge ; facteur de charge ; pH de charge ; ou arrêt de collecte ;
la réception, par l'un ou les plusieurs processeurs, d'un ou plusieurs descripteurs moléculaires descriptifs de la protéine thérapeutique ;
la sélection d'un modèle d'apprentissage machine parmi un groupe constitué (i) d'un modèle d'arbre de régression, (ii) d'un modèle d'amplification de gradient extrême, et (iii) d'un modèle de filet élastique, dans lequel chaque modèle a été entraîné avec un ensemble correspondant de données d'entraînement ;
la prédiction, par l'un ou les plusieurs processeurs, d'un indicateur de performance pour le processus de chromatographie hypothétique, en tant que sortie du modèle d'apprentissage machine, au moins en appliquant l'une ou les plusieurs valeurs de paramètre de processus et l'un ou les plusieurs descripteurs moléculaires en tant qu'entrée pour le modèle d'apprentissage machine, dans lequel le modèle d'arbre de régression est sélectionné pour prédire un premier ensemble d'un ou de plusieurs indicateurs de performance, le modèle d'amplification de gradient extrême est sélectionné pour prédire un deuxième ensemble, différent, d'un ou de plusieurs indicateurs de performance, et le modèle de filet élastique est sélectionné pour un troisième ensemble, différent, d'un ou de plusieurs indicateurs de performance, dans lequel :
le premier ensemble d'un ou de plusieurs indicateurs de performance inclut :

dodécylsulfate de sodium d'électrophorèse capillaire sans pour cent de préparation d'échantillon réduit chaîne légère plus chaîne lourde (nrCE-SDS % LC + HC) ;
dodécylsulfate de sodium d'électrophorèse capillaire avec pour cent de préparation d'échantillon réduit pré-chaîne légère (rCE-SDS % Pre-LC) ;
pour cent de chromatographie par échange de cations Basique (CEX % Basic) ;
pour cent de chromatographie d'exclusion de taille haut poids moléculaire (SEC % HMW) ;
pour cent de chromatographie d'exclusion de taille Principal (SEC % Main) ;
pour cent de chromatographie d'exclusion de taille bas poids moléculaire (SEC % LMW) ;
dodécylsulfate de sodium d'électrophorèse capillaire avec pour cent de préparation d'échantillon réduit chaîne lourde (rCE-SDS % HC) ;
dodécylsulfate de sodium d'électrophorèse capillaire avec pour cent de préparation d'échantillon réduit haut poids moléculaire (rCE-SDS % HMW) ;
dodécylsulfate de sodium d'électrophorèse capillaire avec pour cent de préparation d'échantillon réduit pré-chaîne légère plus chaîne légère_chaîne lourde (rCE-SDS % Pre-LC+LC_HC) ;
conductivité de réserve ; et/ou
dodécylsulfate de sodium d'électrophorèse capillaire sans pour cent de préparation d'échantillon réduit Pré_Pic (nrCE-SDS % Pre-Peak),
le deuxième ensemble d'un ou de plusieurs indicateurs de performance inclut :

pour cent de chromatographie par échange de cations Acidique (CEX % Acidic) ;
pour cent de chromatographie par échange de cations Principal (CEX % Main) ;
rendement d'étape ;
dodécylsulfate de sodium d'électrophorèse capillaire avec pour cent de préparation d'échantillon réduit (rCE-SDS % Main) ;
dodécylsulfate de sodium d'électrophorèse capillaire avec pour cent de préparation d'échantillon réduit (rCE-SDS % LMW) ;
pour cent de concentration isoélectrique capillaire acidique (cIEF % Acidic) ;
pour cent de concentration isoélectrique capillaire basique (cIEF % Basic) ; et/ou

pour cent de concentration isoélectrique capillaire principal (cIEF % Main), et
le troisième ensemble d'un ou de plusieurs indicateurs de performance inclut :

dodécylsulfate de sodium d'électrophorèse capillaire avec pour cent de préparation d'échantillon réduit chaîne légère plus chaîne lourde (rCE-SDS % LC + HC) ; et/ou
dodécylsulfate de sodium d'électrophorèse capillaire avec pour cent de préparation d'échantillon réduit chaîne légère (rCE-SDS % LC) ; et
le fait d'amener, par l'un ou les plusieurs processeurs, un ou les deux parmi (i) l'indicateur de performance prédit, et (ii) une indication du fait que l'indicateur de performance prédit respecte ou non un ou plusieurs critères d'acceptabilité, à être présenté(s) à un utilisateur par l'intermédiaire d'une interface utilisateur.

**2.** Procédé de facilitation de sélection de paramètres de chromatographie pour un processus de purification durant la fabrication d'une protéine thérapeutique, le procédé comprenant :
la réception, par un ou plusieurs processeurs d'un système informatique, d'un ou plusieurs indicateurs de performance associés à un processus de chromatographie hypothétique, dans lequel l'un ou les plusieurs indicateurs de performance peuvent être choisis parmi un premier ensemble d'indicateurs de performance, un deuxième ensemble d'indicateurs de performance, ou un troisième ensemble d'indicateurs de performance, et dans lequel :

le premier ensemble d'indicateurs de performance inclut : dodécylsulfate de sodium d'électrophorèse capillaire sans pour cent de préparation d'échantillon réduit chaîne légère plus chaîne lourde (nrCE-SDS % LC + HC) ;
dodécylsulfate de sodium d'électrophorèse capillaire avec pour cent de préparation d'échantillon réduit pré-chaîne légère (rCE-SDS % Pre-LC) ;
pour cent de chromatographie par échange de cations Basique (CEX % Basic) ;
pour cent de chromatographie d'exclusion de taille haut poids moléculaire (SEC % HMW) ;
pour cent de chromatographie d'exclusion de taille Principal (SEC % Main) ;
pour cent de chromatographie d'exclusion de taille bas poids moléculaire (SEC % LMW) ;
dodécylsulfate de sodium d'électrophorèse capillaire avec pour cent de préparation d'échantillon réduit chaîne lourde (rCE-SDS % HC) ;
dodécylsulfate de sodium d'électrophorèse capillaire avec pour cent de préparation d'échantillon réduit haut poids moléculaire (rCE-SDS % HMW) ;
dodécylsulfate de sodium d'électrophorèse capillaire avec pour cent de préparation d'échantillon réduit pré-chaîne légère plus chaîne légère_chaîne lourde (rCE-SDS % Pre-LC+LC_HC) ;
conductivité de réserve ; et/ou
dodécylsulfate de sodium d'électrophorèse capillaire sans pour cent de préparation d'échantillon réduit Pré_Pic (nrCE-SDS % Pre-Peak),
le deuxième ensemble d'indicateurs de performance inclut :

pour cent de chromatographie par échange de cations Acidique (CEX % Acidic) ;
pour cent de chromatographie par échange de cations Principal (CEX % Main) ;
rendement d'étape ;
dodécylsulfate de sodium d'électrophorèse capillaire avec pour cent de préparation d'échantillon réduit (rCE-SDS % Main) ;
dodécylsulfate de sodium d'électrophorèse capillaire avec pour cent de préparation d'échantillon réduit (rCE-SDS % LMW) ;
pour cent de concentration isoélectrique capillaire acidique (cIEF % Acidic) ;
pour cent de concentration isoélectrique capillaire basique (cIEF % Basic) ; et/ou
pour cent de concentration isoélectrique capillaire principal (cIEF % Main), et
le troisième ensemble d'indicateurs de performance inclut :

dodécylsulfate de sodium d'électrophorèse capillaire avec pour cent de préparation d'échantillon réduit chaîne légère plus chaîne lourde (rCE-SDS % LC + HC) ; et/ou dodécylsulfate de sodium d'électrophorèse capillaire avec pour cent de préparation d'échantillon réduit chaîne légère (rCE-SDS % LC) ;
la réception, par l'un ou les plusieurs processeurs, d'un ou plusieurs descripteurs moléculaires descriptifs de la protéine thérapeutique ;
la sélection d'un modèle d'apprentissage machine parmi un groupe constitué (i) d'un modèle d'arbre de régression, (ii) d'un modèle d'amplification de gradient extrême, et (iii) d'un modèle de filet élastique, dans lequel chaque modèle a été entraîné avec un ensemble correspondant de données d'entraînement ;
la prédiction, par l'un ou les plusieurs processeurs, d'une valeur de paramètre de processus pour un paramètre de processus associé au processus de chromatographie hypothétique, en tant que sortie du modèle d'apprentissage machine, au moins en appliquant l'un ou les plusieurs indicateurs de performance et l'un ou les plusieurs descripteurs moléculaires en tant qu'entrée pour le modèle d'apprentissage machine, dans lequel la valeur de paramètre de processus inclut : pH de tampon ; pH de tampon d'élution ; conductivité de tampon d'élution ; molarité de tampon d'élution ; pente de gradient ; vélocité linéaire ; conductivité de charge ; facteur de charge ; pH de charge ; ou arrêt de collecte, dans lequel l'un ou les plusieurs indicateurs de performance indiquent un aspect de performance du processus de chromatographie hypothétique, dans lequel l'un ou les plusieurs paramètres de processus incluent des paramètres concernant des conditions ou caractéristiques du processus de chromatographie hypothétique, et dans lequel le modèle d'arbre de régression est sélectionné pour prédire la valeur de paramètre de processus lorsque l'un ou les plusieurs indicateurs de performance sont inclus dans le premier ensemble d'indicateurs de performance, le modèle d'amplification de gradient extrême est sélectionné pour prédire le paramètre de processus lorsque l'un ou les plusieurs indicateurs de performance sont inclus dans le deuxième ensemble d'indicateurs de performance, et le modèle de filet élastique est sélectionné pour prédire le paramètre de processus lorsque l'un ou les plusieurs indicateurs de performance sont inclus dans le troisième ensemble d'indicateurs de performance ; et
le fait d'amener, par l'un ou les plusieurs processeurs, une ou les deux parmi (i) la valeur de paramètre de processus prédite, et (ii) une plage de précision prédite de la valeur de paramètre de processus prédite, à être présentée(s) à un utilisateur par l'intermédiaire d'une interface utilisateur.

3. Procédé de la revendication 1 ou 2, dans lequel le processus de chromatographie hypothétique est un processus sélectionné parmi un groupe constitué :

d'un processus de chromatographie par échange de cations (CEX) hypothétique ;
d'un processus de chromatographie par exclusion de taille (SEC) hypothétique ; et
d'un processus de chromatographie de Protéine A.

4. Procédé de l'une quelconque des revendications 1 à 3, comprenant en outre :
la détermination, par l'un ou les plusieurs processeurs, d'au moins un de l'un ou des plusieurs descripteurs moléculaires sur la base d'informations de séquence associées à la protéine thérapeutique.

5. Procédé de l'une quelconque des revendications 1 à 4, comprenant en outre :
la détermination, par l'un ou les plusieurs processeurs, d'au moins un de l'un ou des plusieurs descripteurs moléculaires sur la base d'une mesure expérimentale d'une caractéristique physique de la protéine thérapeutique.

6. Procédé de l'une quelconque des revendications 1 à 5, dans lequel au moins un de l'un ou des plusieurs descripteurs moléculaires est une fonction de niveau de pH.

7. Procédé de l'une quelconque des revendications 1 ou 3 à 6, dans lequel :

l'indicateur de performance inclut SEC % HMW ; et
le procédé comprend en outre les actions suivantes :

prédire, par l'un ou les plusieurs processeurs, un rendement pour le processus de chromatographie hypothétique au moins en analysant des paramètres de processus et des descripteurs moléculaires en utilisant un modèle d'apprentissage machine supplémentaire, dans lequel le modèle d'apprentissage machine supplémentaire est un autre modèle d'amplification de gradient extrême ; et
amener, par l'un ou les plusieurs processeurs, un ou les deux parmi (i) le rendement prédit, et (ii) une indication du fait que le rendement prédit respecte ou non un ou plusieurs critères d'acceptabilité supplémentaires, à être présenté (é) (s) à l'utilisateur par l'intermédiaire de l'interface utilisateur.

8. Procédé de la revendication 1, comprenant en outre la réception de données indicatives d'un indicateur de performance d'intérêt en provenance d'un utilisateur par l'intermédiaire d'une interface utilisateur.

9. Procédé de la revendication 1, comprenant en outre :

la sélection d'une ou plusieurs valeurs de paramètre de processus pour un processus de chromatographie pour la protéine thérapeutique sur la base de l'indicateur de performance présenté et/ou de l'indication présentée ; et
la réalisation du processus de chromatographie pour la protéine thérapeutique selon les valeurs de paramètre de processus sélectionnées.

**10.** Procédé de la revendication 2, comprenant en outre :

la sélection d'une ou plusieurs valeurs de paramètre de processus pour un processus de chromatographie pour la protéine thérapeutique sur la base de la valeur de paramètre de processus prédite présentée, et/ou de la plage de précision prédite ; et
la réalisation du processus de chromatographie pour la protéine thérapeutique selon les valeurs de paramètre de processus sélectionnées.

**11.** Support ou supports non transitoire(s) lisible(s) par ordinateur stockant des instructions qui, lorsqu'elles sont exécutées par un ou plusieurs processeurs d'un système informatique, amènent le système informatique à réaliser le procédé de l'une quelconque des revendications 1 à 8.

**12.** Système informatique, comprenant :

un ou plusieurs processeurs ; et
un ou plusieurs supports non transitoires lisibles par ordinateur stockant des instructions qui, lorsqu'elles sont exécutées par l'un ou les plusieurs processeurs, amènent le système informatique à réaliser le procédé de l'une quelconque des revendications 1 à 8.

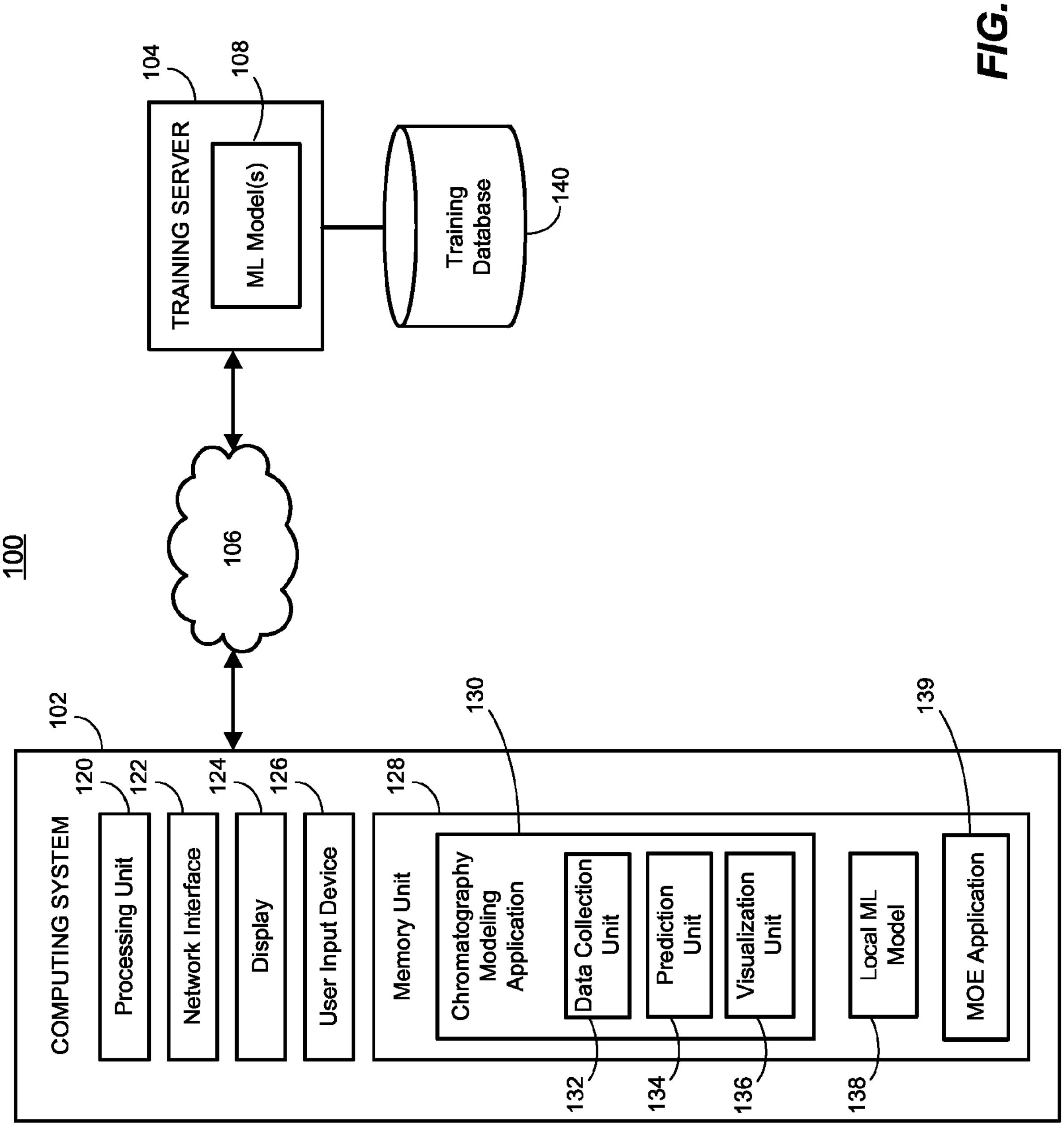
100
COMPUTING SYSTEM
102
Processing Unit
120
Network Interface
122
Display
124
User Input Device
126
Memory Unit
128
Chromatography Modeling Application
130
Data Collection Unit
132
Prediction Unit
134
Visualization Unit
136
Local ML Model
138
MOE Application
139
106
TRAINING SERVER
104
ML Model(s)
108
Training Database
140

FIG. 1

FIG. 2
(prior art)

10

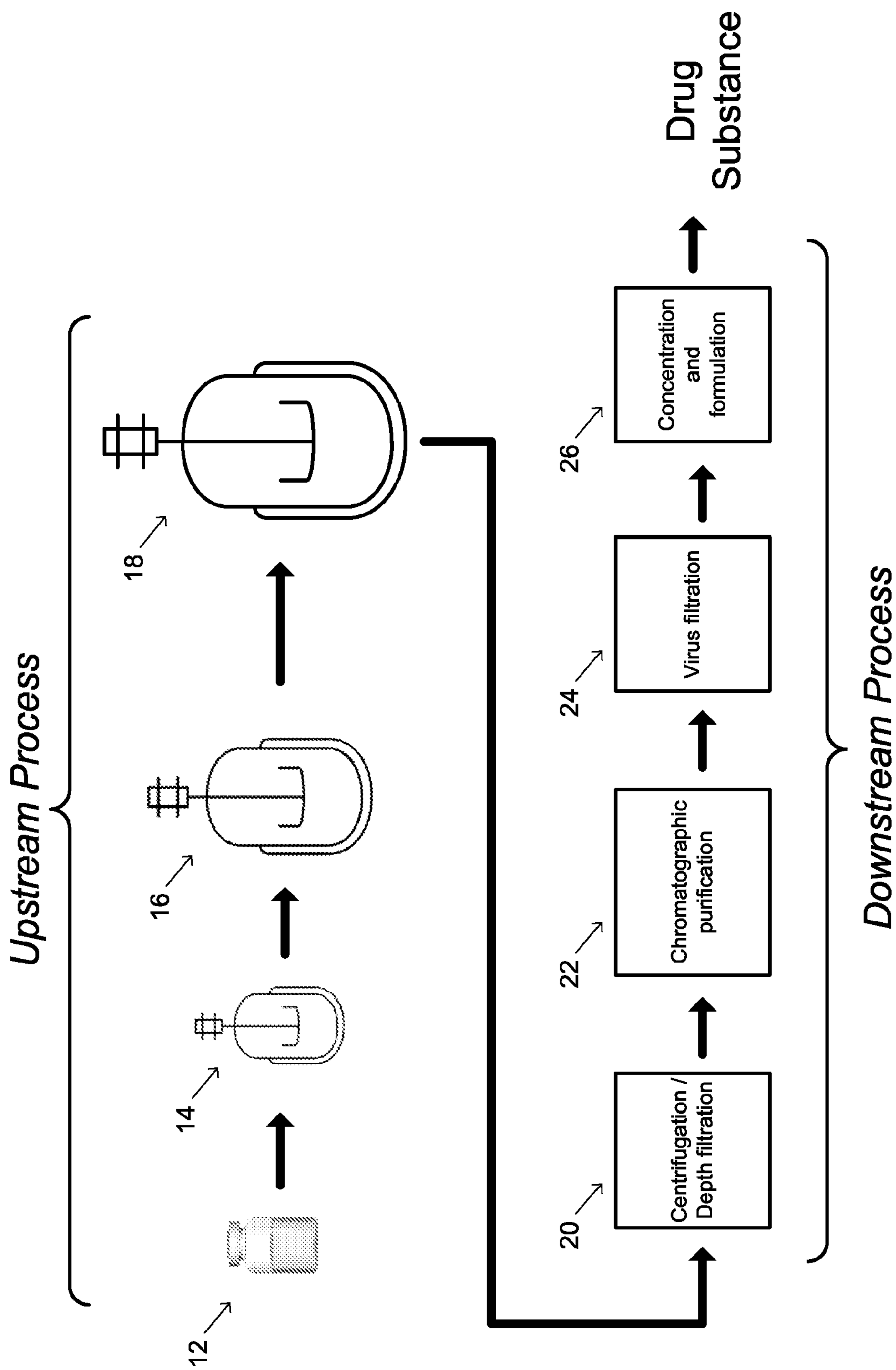

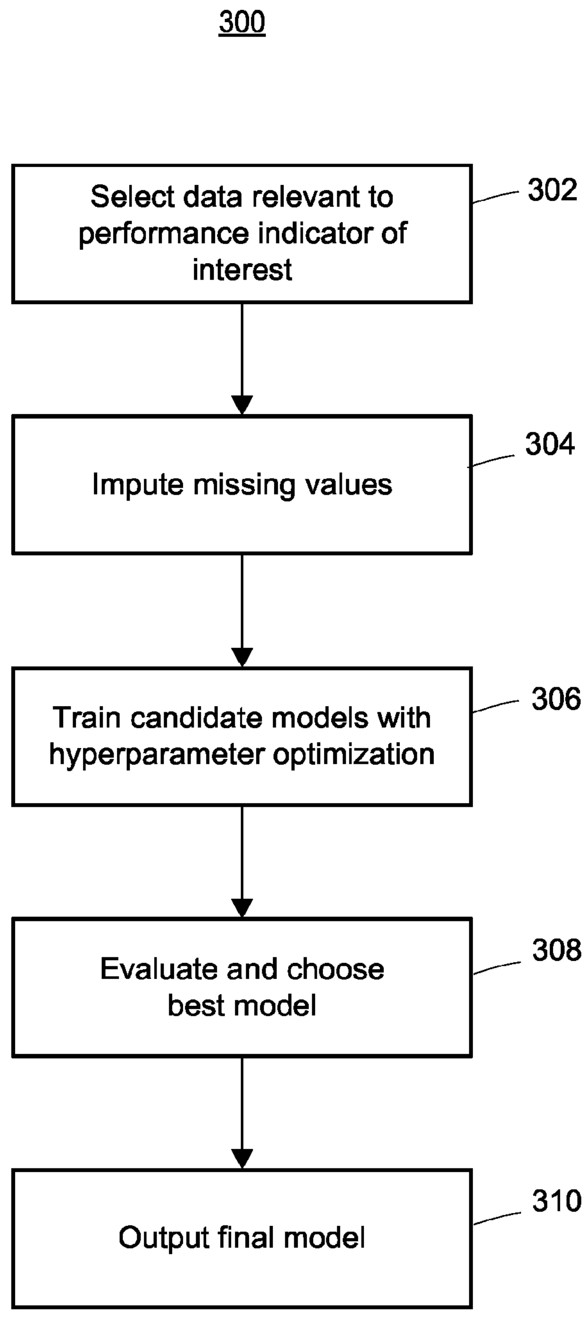
300
Select data relevant to performance indicator of interest
302
Impute missing values
304
Train candidate models with hyperparameter optimization
306
Evaluate and choose best model
308
Output final model
310

FIG. 3

400

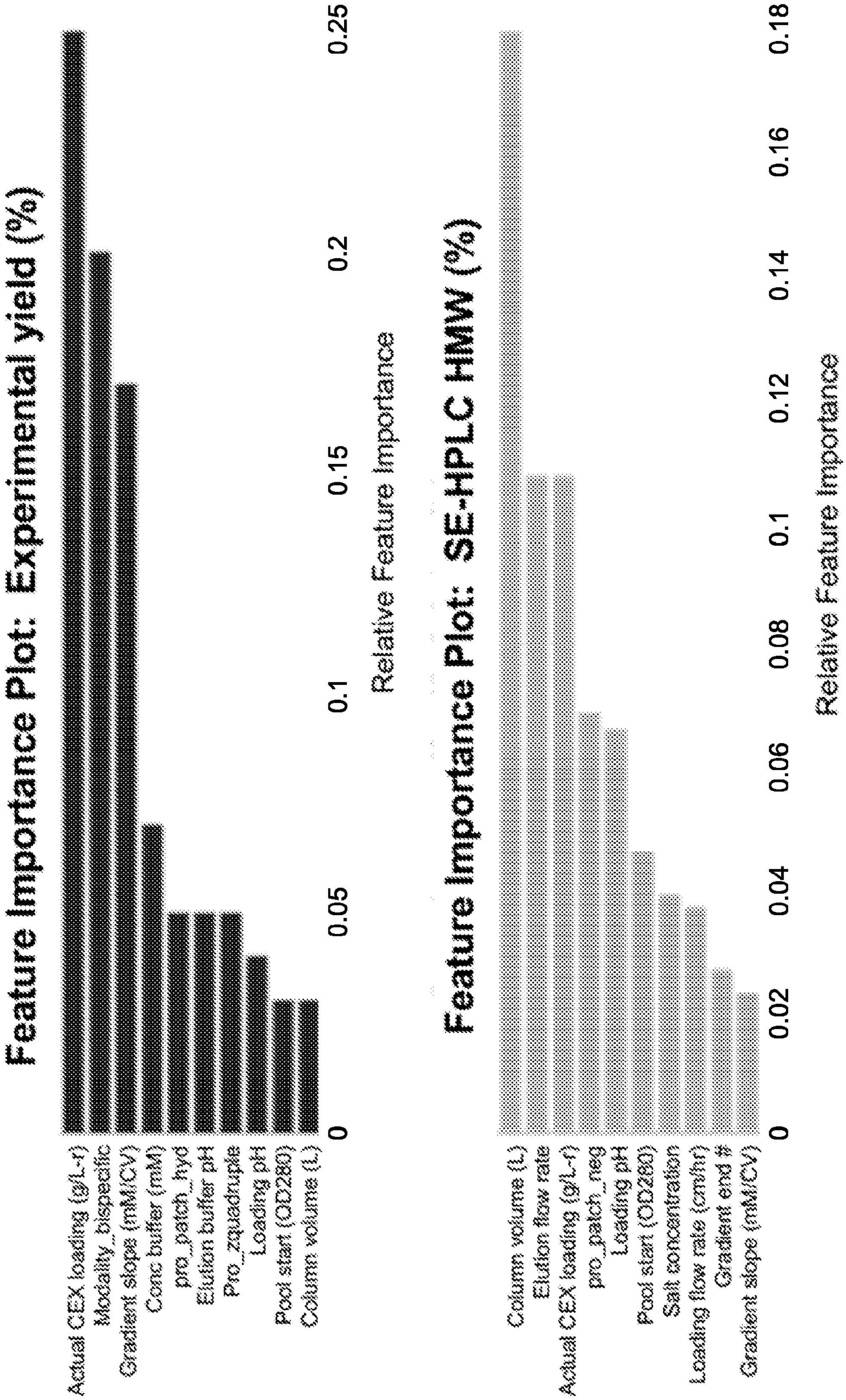

Feature Importance Plot: Experimental yield (%)
Actual CEX loading (g/L-r)
Modality_bispecific
Gradient slope (mM/CV)
Conc buffer (mM)
pro_patch_hyd
Elution buffer pH
Pro_zquadruple
Loading pH
Pool start (OD280)
Column volume (L)
0
0.05
0.1
0.15
0.2
0.25
Relative Feature Importance
Feature Importance Plot: SE-HPLC HMW (%)
Column volume (L)
Elution flow rate
Actual CEX loading (g/L-r)
pro_patch_neg
Loading pH
Pool start (OD280)
Salt concentration
Loading flow rate (cm/hr)
Gradient end #
Gradient slope (mM/CV)
0
0.02
0.04
0.06
0.08
0.1
0.12
0.14
0.16
0.18
Relative Feature Importance


*FIG. 4A*

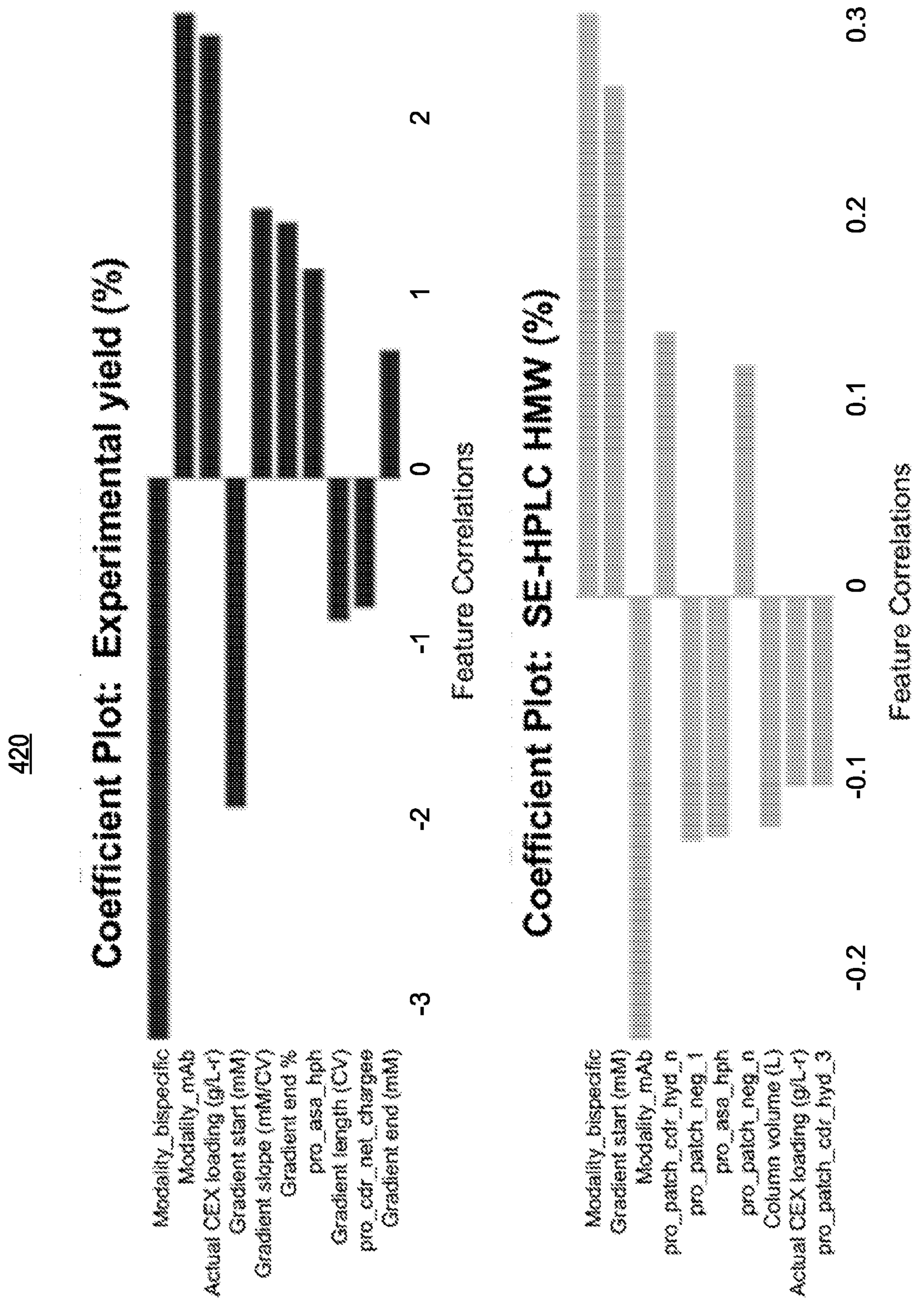
420
Coefficient Plot: Experimental yield (%)
Modality_bispecific
Modality_mAb
Actual CEX loading (g/L-r)
Gradient start (mM)
Gradient slope (mM/CV)
Gradient end %
pro_asa_hph
Gradient length (CV)
pro_cdr_net_charges
Gradient end (mM)
-3
-2
-1
0
1
2
Feature Correlations
Coefficient Plot: SE-HPLC HMW (%)
Modality_bispecific
Gradient start (mM)
Modality_mAb
pro_patch_cdr_hyd_n
pro_patch_neg_1
pro_asa_hph
pro_patch_neg_n
Column volume (L)
Actual CEX loading (g/L-r)
pro_patch_cdr_hyd_3
-0.2
-0.1
0
0.1
0.2
0.3
Feature Correlations

*FIG. 4B*

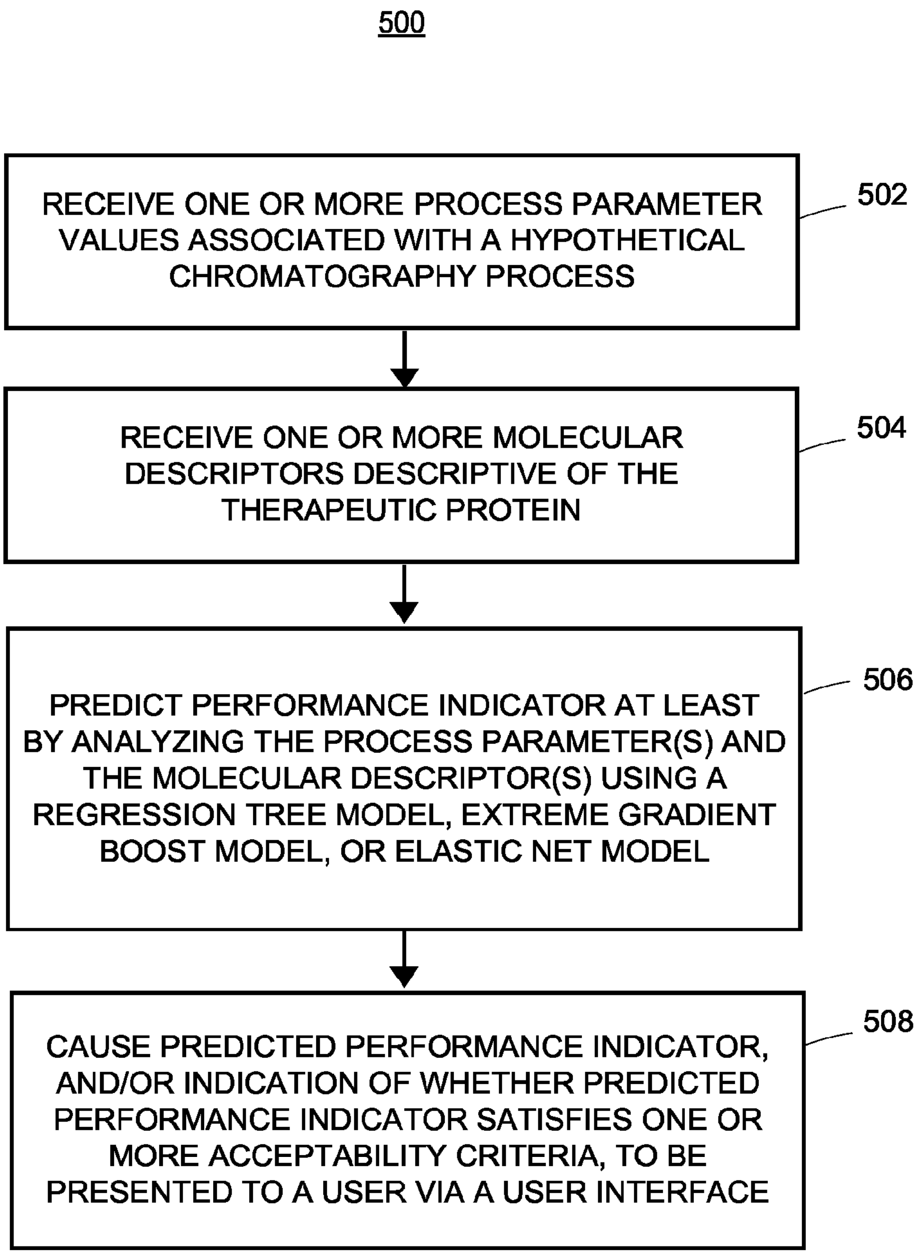
500
RECEIVE ONE OR MORE PROCESS PARAMETER VALUES ASSOCIATED WITH A HYPOTHETICAL CHROMATOGRAPHY PROCESS
502
RECEIVE ONE OR MORE MOLECULAR DESCRIPTORS DESCRIPTIVE OF THE THERAPEUTIC PROTEIN
504
PREDICT PERFORMANCE INDICATOR AT LEAST BY ANALYZING THE PROCESS PARAMETER(S) AND THE MOLECULAR DESCRIPTOR(S) USING A REGRESSION TREE MODEL, EXTREME GRADIENT BOOST MODEL, OR ELASTIC NET MODEL
506
CAUSE PREDICTED PERFORMANCE INDICATOR, AND/OR INDICATION OF WHETHER PREDICTED PERFORMANCE INDICATOR SATISFIES ONE OR MORE ACCEPTABILITY CRITERIA, TO BE PRESENTED TO A USER VIA A USER INTERFACE
508

*FIG. 5A*

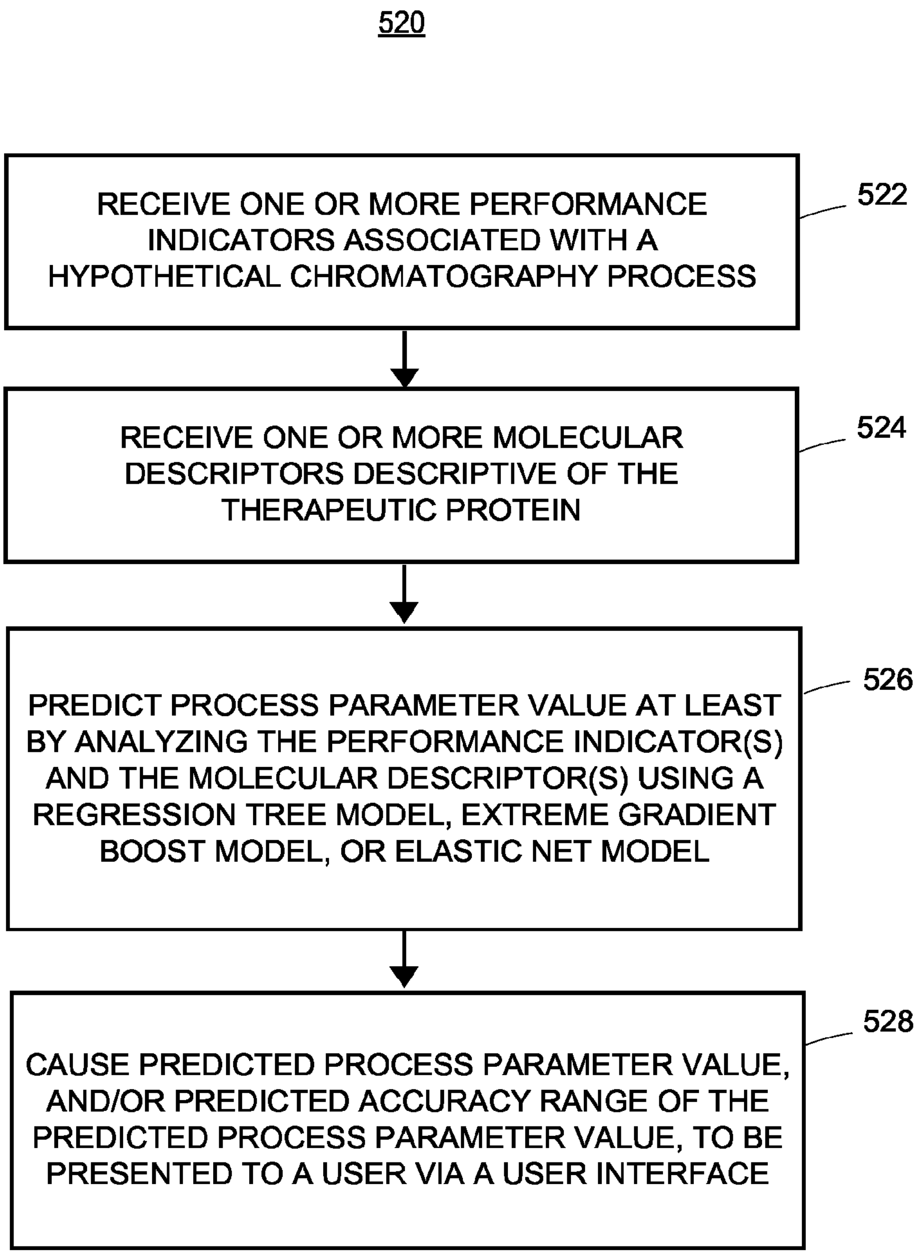
520
RECEIVE ONE OR MORE PERFORMANCE INDICATORS ASSOCIATED WITH A HYPOTHETICAL CHROMATOGRAPHY PROCESS
522
RECEIVE ONE OR MORE MOLECULAR DESCRIPTORS DESCRIPTIVE OF THE THERAPEUTIC PROTEIN
524
PREDICT PROCESS PARAMETER VALUE AT LEAST BY ANALYZING THE PERFORMANCE INDICATOR(S) AND THE MOLECULAR DESCRIPTOR(S) USING A REGRESSION TREE MODEL, EXTREME GRADIENT BOOST MODEL, OR ELASTIC NET MODEL
526
CAUSE PREDICTED PROCESS PARAMETER VALUE, AND/OR PREDICTED ACCURACY RANGE OF THE PREDICTED PROCESS PARAMETER VALUE, TO BE PRESENTED TO A USER VIA A USER INTERFACE
528

*FIG. 5B*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017174580 A1


### Non-patent literature cited in the description


- **TARASOVA IRINA A. et al.** Predictive chromatography of peptides and proteins as a complementary tool for proteomics. *ANALYST*, 01 January 2016, vol. 141 (16), ISSN 0003-2654, 4816-4832
- **SONG MINGHU et al.** Prediction of Protein Retention Times in Anion-Exchange Chromatography Systems Using Support Vector Regression. *JOURNAL OF CHEMICAL INFORMATION AND COMPUTER SCIENCES*, 01 November 2002, vol. 42 (6), ISSN 0095-2338, 1347-1357
- **LIU SONGSONG et al.** Optimal Antibody Purification Strategies Using Data-Driven Models. *ENGINEERING*, 01 December 2019, vol. 5 (6), ISSN 2095-8099, 1077-1092
- **SWANSON RYAN K. et al.** Accounting for host cell protein behavior in anion-exchange chromatography. *BIOTECHNOLOGY PROGRESS*, vol. 32 (6)
- **RYAN K SWANSON et al.** Proteomics-based, multivariate random forest method for prediction of protein separation behavior during cation-exchange chromatography. *JOURNAL OF CHROMATOGRAPHY A*, vol. 1249